# EUROPEAN PATENT SPECIFICATION

(11) **EP 2 134 834 B1**
(45) Date of publication and mention of the grant of the patent: **17.04.2013**
(21) Application number: 08726344.8
(22) Date of filing: 03.03.2008
(51) Int. Cl.: C12N 1/19, C12P 21/02

(54) **PRODUCTION OF GLYCOPROTEINS WITH MODIFIED FUCOSYLATION**
HERSTELLUNG VON GLYCOPROTEINEN MIT MODIFIZIERTER FUCOSYLIERUNG
PRODUCTION DE GLYCOPROTÉINES À FUCOSYLATION MODIFIÉE

(30) Priority: 07.03.2007 US 905345 P
(43) Date of publication of application: 23.12.2009
(73) Proprietor: GlycoFi, Inc., Lebanon, NH 03766 (US)
(72) Inventor: HAMILTON, Stephen, Lebanon, New Hampshire 03766 (US)
(74) Representative: Buchan, Gavin MacNicol
(86) International application number: PCT/US2008/002787
(87) International publication number: WO 2008/112092

(56) References cited:
- US-A1- 2002 137 134
- HAMILTON STEPHEN R ET AL: "Humanization of yeast to produce complex terminally sialylated glycoproteins." SCIENCE (NEW YORK, N.Y.) 8 SEP 2006, vol. 313, no. 5792, 8 September 2006 (2006-09-08), pages 1441-1443, XP002457470 ISSN: 1095-9203
- MA BING ET AL: "Fucosylation in prokaryotes and eukaryotes." GLYCOBIOLOGY DEC 2006, vol. 16, no. 12, December 2006 (2006-12), pages 158R-184R, XP002490822 ISSN: 0959-6658
- WILDT S ET AL: "THE HUMANIZATION OF N-GLYCOSYLATION PATHWAYS IN YEAST" NATURE REVIEWS. MICROBIOLOGY, NATURE PUBLISHING GROUP, GB, vol. 3, no. 2, 1 February 2005 (2005-02-01), pages 119-128, XP009064525 ISSN: 1740-1526
- CHIGIRA YUKO ET AL: "Engineering of a mammalian O-glycosylation pathway in the yeast Saccharomyces cerevisiae: production of O-fucosylated epidermal growth factor domains." GLYCOBIOLOGY APR 2008, vol. 18, no. 4, April 2008 (2008-04), pages 303-314, XP002490819 ISSN: 1460-2423
- HAMILTON STEPHEN R ET AL: "Glycosylation engineering in yeast: the advent of fully humanized yeast." CURRENT OPINION IN BIOTECHNOLOGY OCT 2007, vol. 18, no. 5, October 2007 (2007-10), pages 387-392, XP022350908 ISSN: 0958-1669
- MATTILA P ET AL: "Functional expression of Escherichia coli enzymes synthesizing GDP-L-fucose from inherent GDP-D-mannose in Saccharomyces cerevisiae." GLYCOBIOLOGY OCT 2000, vol. 10, no. 10, October 2000 (2000-10), pages 1041-1047, XP008023410 ISSN: 0959-6658
- PASCHINGER KATHARINA ET AL: "Fucosyltransferase substrate specificity and the order of fucosylation in invertebrates." GLYCOBIOLOGY MAY 2005, vol. 15, no. 5, May 2005 (2005-05), pages 463-474, XP002490823 ISSN: 0959-6658
- BOBROWICZ PIOTR ET AL: "Engineering of an artificial glycosylation pathway blocked in core oligosaccharide assembly in the yeast Pichia pastoris: production of complex humanized glycoproteins with terminal galactose." GLYCOBIOLOGY SEP 2004, vol. 14, no. 9, September 2004 (2004-09), pages 757-766, XP002354412 ISSN: 0959-6658
- WANG Y ET AL: "Modification of epidermal growth factor-like repeats with O-fucose. Molecular cloning and expression of a novel GDP-fucose protein O-fucosyltransferase." THE JOURNAL OF BIOLOGICAL CHEMISTRY 26 OCT 2001, vol. 276, no. 43, 26 October 2001 (2001-10-26), pages 40338-40345, XP002490867 ISSN: 0021-9258

## Description

### BACKGROUND OF THE INVENTION

### (1) Field of the Invention

The present invention relates to the field of glycobiology, and in particular to methods for genetically engineering host cells that lack an endogenous pathway for fucosylating *N*-glycans of glycoproteins to be able to produce glycoproteins with fucosylated *N*-glycans.

### (2) Description of Related Art

Therapeutic proteins intended for use in humans that are glycosylated should have complex, human *N*-glycosylation patterns. In general, it would be advantageous to produce therapeutic proteins using bacterial or eukaryotic microorganisms because of (a) the ability to rapidly produce high concentrations of protein; (b) the ability to use sterile, well-controlled production conditions (for example, GMP conditions); (c) the ability to use simple, chemically defined growth media; (d) ease of genetic manipulation; (e) the absence of contaminating human or animal pathogens; (f) the ability to express a wide variety of proteins, including those poorly expressed in cell culture owing to toxicity etc.; and, (g) ease of protein recovery (for example, via secretion into the medium). However, prokaryotes and lower eukaryotes do not normally produced proteins having complex *N*-glycosylation patterns. Therefore, animal cells are generally used to produce therapeutic proteins where it is desirable that the protein have a complex, human-like *N*-glycosylation pattern. But, there are a number of significant drawbacks to using animal cells for producing therapeutic proteins.

Only certain therapeutic proteins are suitable for expression in animal cells (for example, those lacking in any cytotoxic effect or other effect adverse to growth). Animal cell culture systems are usually very slow, frequently requiring over a week of growth under carefully controlled conditions to produce any useful quantity of the protein of interest. Protein yields nonetheless compare unfavorably with those from microbial fermentation processes. In addition, cell culture systems typically require complex and expensive nutrients and cofactors, such as bovine fetal serum. Furthermore, growth may be limited by programmed cell death (apoptosis).

Moreover, animal cells (particularly mammalian cells) are highly susceptible to viral infection or contamination. In some cases the virus or other infectious agent may compromise the growth of the culture, while in other cases the agent may be a human pathogen rendering the therapeutic protein product unfit for its intended use. Furthermore, many cell culture processes require the use of complex, temperature-sensitive, animal-derived growth media components, which may carry pathogens such as bovine spongiform encephalopathy

(BSE) prions. Such pathogens are difficult to detect and/or difficult to remove or sterilize without compromising the growth medium. In any case, use of animal cells to produce therapeutic proteins necessitates costly quality controls to assure product safety.

Recently, it has been shown that lower eukaryotes, particularly yeast, can be genetically modified so that they express proteins having complex N-glycosylation patterns that are human-like or humanized. Such genetically modified lower eukaryotes can be achieved by eliminating selected endogenous glycosylation enzymes that are involved in producing high mannose N-glycans and introducing various combinations of exogenous enzymes involved in making complex N-glycans. Methods for genetically engineering yeast to produce complex N-glycans has been described in U.S. Patent No. 7,029,872 and U.S. Published patent Application Nos. 2004/0018590, 2005/0170452, 2006/0286637, 2004/0230042, 2005/0208617, 2004/0171826, 2005/0208617, and 2006/0160179. For example, a host cell can be selected or engineered to be depleted in 1,6-mannosyl transferase activities, which would otherwise add mannose residues onto the N-glycan on a glycoprotein, and then further engineered to include each of the enzymes involved in producing complex, human-like N-glycans.

Animal and human cells have a fucosyltransferase pathway that adds a fucose residue to the GlcNAc residue at the reducing end the N-glycans on a protein. The fucosylation pathway in humans consists of a GDP-mannose dehydratase and GDP-keto-deoxy-mannose-epimerase/GDP-keto-deoxy-galactose-reductase (FX protein), both located in the cytoplasm, which in concert converts GDP-mannose to GDP-fucose; a GDP-fucose transporter located in the membrane of the Golgi apparatus, which transports the GDP-fucose into the Golgi apparatus; and a fucosyltransferase (Fut8), which transfers the fucose residue by means of an 1,6-linkage to the 6 position of the GlcNAc residue at the reducing end of the *N*-glycan. In contrast to higher eukaryotes, many lower eukaryotes, for example yeast, lack the enzymes involved in the fucosyltransferase pathway, produce glycoproteins that do not contain fucose (*See* for example, Bretthauer/Catellino, Biotechnol. Appl. Biochem. 30: 193-200 (1999); Rabina et al., Anal. Biochem. 286: 173-178 (2000)). However, the lack of fucose on glycoproteins has been shown to have advantages in certain cases. For example, in the production of monoclonal antibodies, immunoglobulin molecules, and related molecules, it has been shown that removal of the fucose sugar from the *N*-glycan of immunoglobulins increases or alters its binding to selected Ig receptors, which effects changes in properties such as antibody-dependent cellular cytotoxicity, or ADCC. (*See,* for example, U.S. Published Patent Application Nos. 2005/0276805 and US2003/0157108)

However, while removal of fucose from the *N*-glycans of immunoglobulins appears to enhance ADCC activity of the immunoglobulins, fucosylated *N*-glycans appear to be important for other glycoproteins. For example, the deletion of the fucosyltransferase gene in mice induces severe growth retardation, early death during post-natal development, and emphysema-like changes in the lung. These *Fut8*^{-/-} null mice were rescued from the emphysema-like phenotype by administration of exogenous TGF-betal. Additionally, impaired receptor-mediated signaling was rescued by reintroduction of the Fut8 gene, showing that core fucosylation is crucial for proper functioning of growth factor receptors such as TGF-betal and EGF (Wang et al., Meth. Enzymol. 417: 11-22 (2006). In lung tissue derived from *Fut8*^{-/-} mice, the loss of core fucosylation impairs the function of low-density lipoprotein (LDL) receptor-related protein-1 (LRP-1), resulting in a reduction in the endocytosis of insulin like growth factor (IGF)-binding protein-3 (IGFBP-3) (Lee et al., J. Biochem. (Tokyo) 139: 391-8 (2006). In *Fut8-*/- mouse embryonic fibroblast cells, α3β1 integrin-mediated cell migration is abolished and cell signaling is decreased, identifying the core fucose as essential for protein function (Zhao et al., J. Biol. Chem., 281: 38343-38350 (2006). In addition, there may be situations where it is desirable to produce antibody compositions where at least a portion of the antibodies in the compositions are fucosylated in order to decrease ADCC activity. Therefore, in particular cases it will be advantageous to provide lower eukaryotic organisms and cells capable of producing fucosylated glycopeptides. Accordingly, development of methods and materials for the production of lower eukaryotic host cells, such as fungi and yeast, and particularly yeasts such as *Pichia pastoris, K. lactis*, and others, would facilitate development of genetically enhanced yeast strains for the recombinant production of fucosylated glycoproteins.

US-A-20020137134 (Tillman U. Gemgross) discloses expression in a fungal host of a fusion protein between a catalytic domain of a fucosyltransferase and a targeting signal peptide wherein the enzyme is targeted to the endosomal network. The localization signal may be from a mannosyltransferase.

Ma et al (Glycobiology, 16(12) 158R-184R, 2006) reviews fucosylation in prokaryotes and eukaryotes and indicates that the *de novo* synthesis of GDP-fuc requires the enzymes GMD and FX. Subsequently GFTr imports a GDP-Fuc into the Golgi lumen where Golgi-localised FucTs use GDP-Fuc as a substrate. There is a general statement of interest in remodeling the glycosylation patterns in glycoprotein drugs, but nothing is said about how this may be achieved in practice.

Hamilton et al (Science, 313, 1441-1443, 2006) teaches *P.pastoris* which is humanised to eliminate yeast-specific glycosylation and to confer the ability to produce human-like complex terminally sialylated glycoproteins.

### BRIEF SUMMARY OF THE INVENTION

Accordingly, the present invention provides a recombinant *Pichia pastoris* host cell comprising a fucosylation pathway wherein the enzymes and activities comprising the pathway are provided by nucleic acids encoding a GDP-mannose-dehydratase (GMD), a GDP-keto-deoxy-mannose-epimerase/GDP-keto-deoxy-galactose-reductase (FX), a GDP-Fucose transporter (GFTr), and a fusion protein that comprises the catalytic domain of α1,6-fucosyltransferase EC 2.4.1.68 fused to amino acids 1-36 of the *S. cerevisiae Mnn2.*

In further aspects, the host cell further does not display α1,6 mannosyltransferase activity with respect to the *N*-glycan on a glycoprotein and includes an α1,2-mannosidase catalytic, domain fused to a cellular targeting signal peptide not normally associated with the the host cell whereby, upon passage of recombinant glycoprotein through the ER or Golgi apparatus of the host cell, a recombinant glycoprotein comprising a fucosylated Man₅GlcNAc₂ glycoform is produced.

In further aspects, the above host cell further includes a GlcNAc transferase I catalytic domain fused to a cellular targeting signal peptide not normally associated with the catalytic domain and selected to target GlcNAc transferase I activity to the ER or Golgi apparatus of the host cell ; whereby, upon passage of the recombinant glycoprotein through the ER or Golgi apparatus of the host cell, a recombinant glycoprotein comprising a fucosylated GlcNAcMan₅GlcNAc₂glycoform is produced.

In further aspects, the above host cell further includes a mannosidase II catalytic domain fused to a cellular targeting signal peptide not normally associated with the catalytic domain and selected to target mannosidase II activity to the ER or Golgi apparatus of the host cell; whereby, upon passage of the recombinant glycoprotein through the ER or Golgi apparatus of the host cell, a recombinant glycoprotein comprising a fucosylated GlcNAcMan₃GlcNAc₂ glycoform is produced.

In further aspects, the above host cell further includes a GlcNAc, transferase II catalytic domain fused to a cellular targeting signal peptide not normally associated with the catalytic domain and selected to target GlcNAc transferase II activity to the ER or Golgi apparatus of the host cell; thereby, upon passage of the recombinant glycoprotein through the ER or Golgi apparatus of the host cell, a recombinant glycoprotein comprising a fucosylated GlcNAc₂Man₃GlcNAc₂ glycoform is produced.

In further aspects, the above host cell further includes a Galactose transferase II catalytic domain fused to a cellular targeting signal peptide not normally associated with the catalytic domain and; selected to target Galactose transferase II activity to the ER or Golgi apparatus of the host cell, whereby upon passage of the recombinant glycoprotein through the ER or Golgi apparatus of the host cell a recombinant glycol protein comprising a fucosylated Gal₂GlcNAc₂Man₃GlcNAc₂ glycoform is produced.

In further aspects, the above host cell further includes a sialytransferase catalytic domain fused to a cellular targeting signal peptide not normally associated with the catalytic domain and selected to target sialytransferase activity to the ER or Golgi apparatus of the host cell; whereby, upon passage of the recombinant glycoprotein through the ER or Golgi apparatus of the host cell, a recombinant glycoprotein comprising a fucosylated NANA₂Gal₂GlcNAc₂Man₃GlcNAc₂ glycoform is produced.

Transforming the above host cells with a nucleic acid encoding a particular glycoprotein, compositions of the glycoprotein can be produced that comprise a plurality of glycoforms, each glycoform comprising at least one *N*-glycan attached thereto, wherein the glycoprotein composition thereby comprises a plurality of *N*-glycans in which a predominant glycoform comprises a desired fucosylated *N*-glycan. Depending upon the specific glycoprotein desired, the methods of the present invention can be used to obtain glycoprotein compositions in which the predominant *N*-glycoform is present in an amount between 5 and 80 mole percent greater than the next most predominant *N*-glycoform; in further embodiments, the predominant *N*-glycoform may be present in an amount between 10 and 40 mole percent; 20 and 50 mole percent; 30 and 60 mole percent; 40 and 70 mole percent; 50 and 80 mole percent greater than the next most predominant *N*-glycoform. In other embodiments, the predominant *N*-glycoform is a desired fucosylated *N*-glycoform and is present in an amount of greater than 25 mole percent; greater than 35 mole percent; greater than 50 mole percent; greater than 60 mole percent; or greater than 75 mole percent of the total number of *N*-glycans.

Thus, are provided host cells for producing glycoprotein compositions comprising a plurality of glycoforms, each glycoform comprising at least one *N*-glycan attached thereto, wherein the glycoprotein composition thereby comprises a plurality of fucosylated *N*-glycans in which the predominant *N*-glycan is selected from the group consisting of Man₅GlcNAc₂, GlcNAcMan₅GlcNAc₂, Man₃GlcNAc₂, GlcNAcMan₃GlcNAc₂, GlcNAc₂Man₃GlcNAc₂, GalGlcNAc₂Man₃GlcNAc₂; Gal₂GlcNAc₂Man₃GlcNAc₂, NANAGal₂GlcNAc₂Man₃GlcNAc₂, and NANA₂Gal₂GlcNAc₂Man₃GlcNAc₂.

In further aspects, greater than 25 mole percent of the plurality of fucosylated *N-*glycans consists essentially of a fucosylated glycoform in which the glycoform is selected from the group consisting of Man₅GlcNAc₂, GlcNAcMan₅GlcNAc₂, Man₃GlcNAc₂, GlcNAcMan₃GlcNAc₂, GlcNAc₂Man₃GlcNAc₂, GalGlcNAc₂Man₃GlcNAc₂, Gal₂GlcNAc₂Man₃GlcNAc₂, NANAGal₂GlcNAc₂Man₃GlcNAc₂, and NANA₂Gal₂GlcNAc₂Man₃GlcNAC₂.

In further still aspects, greater than 25 mole percent; greater than 35 mole percent; greater than 50 mole percent; greater than 60 mole percent; greater than 75 mole percent; or greater than 90 mole percent of the plurality of *N*-glycans consists essentially of a fucosylated glycoform in which the glycoform is selected from the group consisting of Man₅GlcNAc₂, GlcNAcMan₅GlcNAc₂, Man₃GlcNAc₂, GlcNAcMan₃GlcNAc₂, GlcNAc₂Man₃GlcNAc₂, GalGlcNAc₂Man₃GlcNAc₂, Gal₂GlcNAc₂Man₃GlcNAc₂, NANAGal₂GlcNAc₂Man₃GlcNAc₂, and NANA₂Gal₂GlcNAc₂Man₃GlcNAc₂.

In the above glycoprotein composition, the fucose is in an α1,3-linkage with the GlcNAc at the reducing end of the *N*-glycan, an α1,6-linkage with the GlcNAc at the reducing end of the *N*-glycan, an α1,2-linkage with the Gal at the non-reducing end of the *N*-glycan, an α1,3-linkage with the GlcNac at the non-reducing end of the *N*-glycan, or an α1,4-linkage with a GlcNAc at the non-reducing end of the *N*-glycan.

Therefore, in particular aspects of the above the glycoprotein compositions, the glycoform is in an α1,3-linkage or α1,6-linkage fucose to produce a glycoform selected from the group consisting of Man₅GlcNAc₂(Fuc), GlcNAcMan₅GlcNAc₂(Fuc), Man₃GlcNAc₂(Fuc), GlcNAcMan₃GlcNAc₂(Fuc), GlcNAc₂Man₃GlcNAc₂(Fuc), GalGlcNAc₂Man₃GlcNAc₂(Fuc), Gal₂GlcNAc₂Man₃GlcNAc₂(Fuc), NANAGal₂GlcNAc₂Man₃GlcNAc₂(Fuc), and NANA₂Gal₂GlcNAc₂Man₃GlcNAc₂(Fuc); in an α1,3-linkage or α1,4-linkage fucose to produce a glycoform selected from the group consisting of GlcNAc(Fuc)Man₅GlcNAc₂, GlcNAc(Fuc)Man₃GlcNAc₂, GlcNAc₂(Fuc_{1.2})Man₃GlcNAc₂, GalGlcNAc₂(Fuc₁₋₂)Man₃GlcNAc₂, Gal₂GlcNAc₂(Fuc₁₋₂)Man₃GlcNAc₂, NANAGal₂GlcNAc₂(Fuc₁₋. ₂)Man₃GlcNAc₂, and NANA₂Gal₂GlcNAc₂(Fuc₁₋₂)Man₃GlcNAc₂; or in an α1,2-linkage fucose to produce a glycoform selected from the group consisting of Gal(Fuc)GlcNAc₂Man₃GlcNAc₂, Gal₂(Fuc₁₋₂)GlcNAc₂Man₃GlcNAc₂, NANAGal₂(Fuc₁₋₂)GlcNAc₂Man₃GlcNAc₂, and NANA₂Gal₂(Fuc₁₋₂)GlcNAc₂Man₃GlcNAc₂.

In other aspects, the glycoprotein composition of the present invention comprise compositions wherein the above *N*-glycoform is present at a level from about 5 to 80 mole percent; 10 to 40 mole percent; 20 to 50 mole percent; 30 to 60 mole percent; 40 to 70 mole percent; or 50 to 80 mole percent greater than the next most predominant *N*-glycoform.

### Definitions

As used herein, the terms "*N*-glycan" and "glycoform" are used interchangeably and refer to an *N*-linked oligosaccharide, for example, one that is attached by an asparagine-*N-*acetylglucosamine linkage to an asparagine residue of a polypeptide. *N*-linked glycoproteins contain an *N*-acetylglucosamine residue linked to the amide nitrogen of an asparagine residue in the protein. The predominant sugars found on glycoproteins are glucose (Glc), galactose (Gal), mannose (Man), fucose (Fuc), *N*-acetylgalactosamine (GalNAc), *N*-acetylglucosamine (GlcNAc) and sialic acid (for example, *N*-acetyl-neuraminic acid (NANA)). The processing of the sugar groups occurs co-translationally in the lumen of the ER and continues in the Golgi apparatus for *N*-linked glycoproteins.

*N*-glycans have a common pentasaccharide core of Man₃GlcNAc₂. *N*-glycans differ with respect to the number of branches (antennae) comprising peripheral sugars (for example, GlcNAc, galactose, fucose, and sialic acid) that are added to the Man₃GlcNAc₂ core structure which is also referred to as the "trimannose core", the "pentasaccharide core", or the "paucimannose core". *N*-glycans are classified according to their branched constituents (for example, high mannose, complex or hybrid). A "high mannose" type *N*-glycan has five or more mannose residues. A "complex" type *N*-glycan typically has at least one GlcNAc attached to the 1,3 mannose arm and at least one GlcNAc attached to the 1,6 mannose arm of a "trimannose" core. Complex *N*-glycans may also have galactose or *N*-acetylgalactosamine residues that are optionally modified with sialic acid or derivatives (for example, "NANA" or "NeuAc", where "Neu" refers to neuraminic acid and "Ac" refers to acetyl). Complex *N*-glycans may also have intrachain substitutions comprising "bisecting" GlcNAc and core fucose ("Fuc"). As an example, when a *N*-glycan comprises a bisecting GlcNAc on the trimannose core, the structure can be represented as Man₃GlcNAc₂(GlcNAc) or Man₃GlcNAc3. When an *N*-glycan comprises a core fucose attached to the trimannose core, the structure may be represented as Man₃GlcNAc₂(Fuc). Complex *N*-glycans may also have multiple antennae on the "trimannose core," often referred to as "multiple antennary glycans." A "hybrid" *N*-glycan has at least one GlcNAc on the terminal of the 1,3 mannose arm of the trimannose core and zero or more mannoses on the 1,6 mannose arm of the trimannose core. The various N-glycans are also referred to as "glycoforms."

Abbreviations used herein are of common usage in the art, see, for example, abbreviations of sugars, above. Other common abbreviations include "PNGase", or "glycanase" or "glucosidase" which all refer to peptide *N*-glycosidase F (EC 3.2.2.18).

The term "expression control sequence" as used herein refers to polynucleotide sequences that are necessary to affect the expression of coding sequences to which they are operatively linked. Expression control sequences are sequences that control the transcription, post-transcriptional events, and translation of nucleic acid sequences. Expression control sequences include appropriate transcription initiation, termination, promoter, and enhancer sequences; efficient RNA processing signals such as splicing and polyadenylation signals; sequences that stabilize cytoplasmic mRNA; sequences that enhance translation efficiency (for example, ribosome binding sites); sequences that enhance protein stability; and when desired, sequences that enhance protein secretion. The nature of such control sequences differs depending upon the host organism; in prokaryotes, such control sequences generally include promoter, ribosomal binding site, and transcription termination sequence. The term "control sequences" is intended to include, at a minimum, all components whose presence is necessary for expression, and can also include additional components whose presence is advantageous, for example, leader sequences and fusion partner sequences.

The term "recombinant host cell" ("expression host cell", "expression host system", "expression system" or simply "host cell"), as used herein, is intended to refer to a cell into which a recombinant vector has been introduced. It should be understood that such terms are intended to refer not only to the particular subject cell but to the progeny of such a cell. Because certain modifications may occur in succeeding generations due to either mutation or environmental influences, such progeny may not, in fact, be identical to the parent cell, but are still included within the scope of the term "host cell" as used herein. A recombinant host cell may be an isolated cell or cell line grown in culture or may be a cell which resides in a living tissue or organism.

The term "eukaryotic" refers to a nucleated cell or organism, and includes insect cells, plant cells, mammalian cells, animal cells, and lower eukaryotic cells.

The term "lower eukaryotic cells" includes yeast, fungi, collar-flagellates, microsporidia, alveolates (for example, dinoflagellates), stramenopiles (for example, brown algae, protozoa), rhodophyta (for example, red algae), plants (for example, green algae, plant cells, moss) and other protists. Yeast and fungi include, but are not limited to: *Pichia pastoris, Pichia finlandica, Pichia trehalophila, Pichia koclamae, Pichia membranaefaciens, Pichia minuta (Ogataea minuta, Pichia lindneri), Pichia opuntiae, Pichia thermotolerans, Pichia salictaria, Pichia guercuum, Pichia pijperi, Pichia stiptis, Pichia methanolica, Pichia* sp., *Saccharomyces cerevisiae, Saccharomyces* sp., *Hansenula polymorpha, Kluyveromyces sp., Kluyveromyces lactis, Candida albicans, Aspergillus nidulans, Aspergillus niger, Aspergillus oryzae, Trichoderma reesei, Chrysosporium lucknowense, Fusarium* sp., *Fusarium gramineum, Fusarium venenatum, Physcomitrella patens* and *Neurospora crassa. Pichia sp.,* any *Saccharomyces sp., Hansenula polymorpha,* any *Kluyveromyces sp., Candida albicans,* any *Aspergillus sp., Trichoderma reesei, Chrysosporium lucknowense,* any *Fusarium sp.,* and *Neurospora crassa.*

The term "peptide" as used herein refers to a short polypeptide, for example, one that is typically less than about 50 amino acids long and more typically less than about 30 amino acids long. The term as used herein encompasses analogs and mimetics that mimic structural and thus biological function.

As used herein, the term "predominantly" or variations such as "the predominant" or "which is predominant" will be understood to mean the glycan species that has the highest mole percent (%) of total *N*-glycans after the glycoprotein has been treated with PNGase and released glycans analyzed by mass spectroscopy, for example, MALDI-TOF MS. In other words, the phrase "predominantly" is defined as an individual entity, such as a specific glycoform, is present in greater mole percent than any other individual entity. For example, if a composition consists of species A in 40 mole percent, species B in 35 mole percent and species C in 25 mole percent, the composition comprises predominantly species A.

Unless otherwise defined, all technical and scientific terms used herein have the same meaning as commonly understood by one of ordinary skill in the art to which this Invention pertains. Exemplary methods and materials are described below, although methods and materials similar or equivalent to those described herein can also be used in the practice of the present invention and will be apparent to those of skill in the art. In case of conflit, the present specification, including definitions, will control.

### BRIEF DESCRIPTION OF THE DRAWINGS

Figure 1 illustrates the fucosylation pathway present in many higher eukaryotic cells.

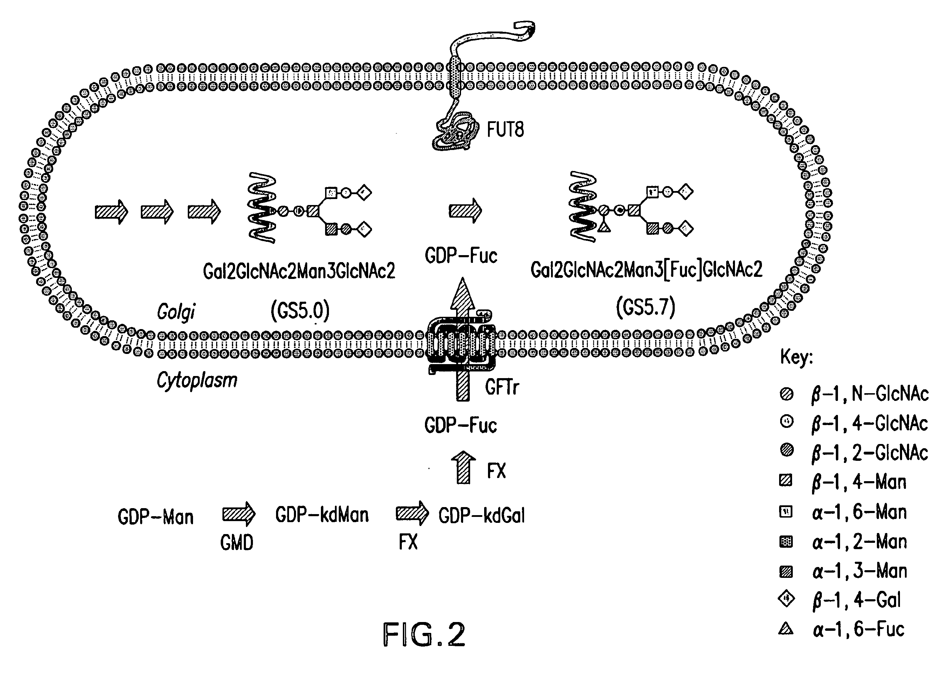

Figure 2 shows the glyco-engineering steps required to produce a recombinant yeast capable of producing fucosylated glycoprotein. Endogenous GDP-Mannose, present in the yeast cytoplasm, is converted to GDP-Fucose by GDP-mannose-dehydratase (GMD) and the bifunctional enzyme FX. Subsequently, the product is translocated into the Golgi apparatus by the GDP-Fucose transporter (GFTr) and fucose is transferred onto the acceptor glycan by α-1,6 fucosyltransferase (*FUT8*). Enzymes are indicated by blue text and metabolic intermediates by black text. GDP-kdMan (GDP-4-keto-6-deoxy-mannose) and GDP-kdGal (GDP-4-keto=6= deoxy-galactose) are intermediates in the conversion GDP mannose to GDP-fucose
Figure 3A shows the vectors used in engineering yeast strains to produce fucosylated glycoproteins. Represented is the expression vector pSH995 into which the fucose biosynthetic and transfer genes are introduced. Introduction of the genes required for biosynthesis and transfer of fucose into pSH995 produced the vector pSH1022:
Figure 3B shows the vector pSH1022. Shown in (B) are the flanking regions of the *TRP2* loci used to integrate the genes into the *Pichia* genome; the dominant selection marker NATr; the GAPDH-CYC expression cassette; and the pUC19 plasmid backbone.
Figure 4A shows a MALDI-TOFscan of the *N*-glycans released from the rat EPO demonstrating that *Pichia pastoris* strain YSH661 (strain RDP974 transformed with vector pSH1022 containing the fucosylation pathway genes) produced rEPO comprising Gal₂GlcNAc₂Man₃GlcNAc₂(Fuc) and Gal₂GlcNAc₂Man₃GlcNAc₂ *N*-glycans. The Gal₂GlcNAc₂Man₃GlcNAc₂(Fuc) *N*-glycans are within the box.
Figure 4B shows a MALDI-TOF scan of the *N*-glycans released from the rat EPO control strain YSH660 (strain RDP974 transformed with control vector pSH995) produced afucosylated or fucose-free Gal₂GlcNAc₂Man₃GlcNA_{C2} *N*-glycans only.

### DETAILED DESCRIPTION OF THE INVENTION

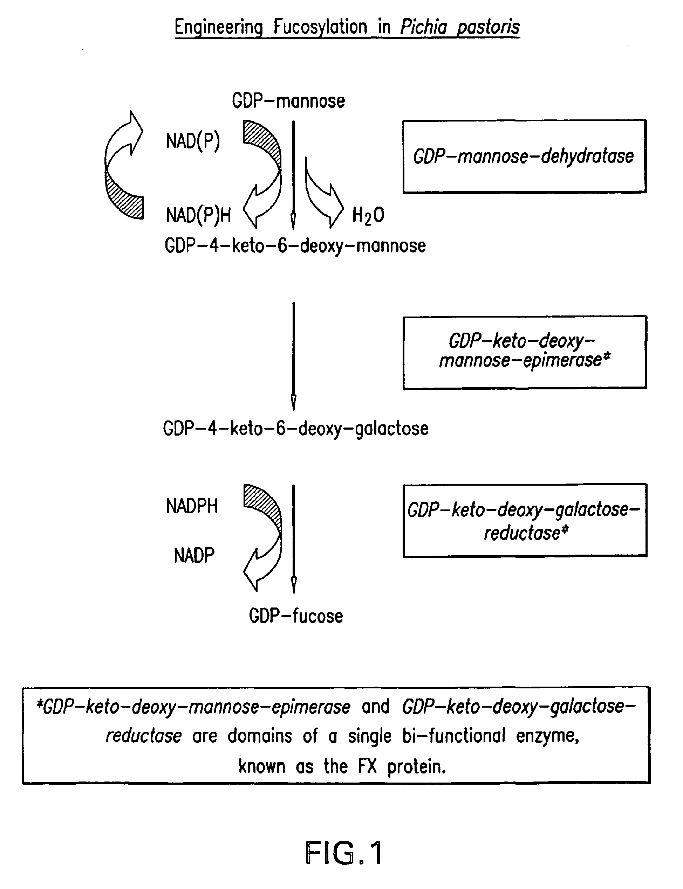

The present invention provides methods and materials for the genetically engineering host cells capable of producing glycoproteins proteins that have fucosylated *N-*glycans. An overview of the fucosylation pathway in higher eukaryotes is shown in Figure 1.

GDP-mannose-4,6-dehydratase (GMD) (EC 4.2.1.47) converts QDP-mannose to GDP-4-keto-6'-deoxy-mannose in the presence of NAD has been identified in a number of species. The human GMP(hGMD) is encoded by the nucleotide sequence shown in SEQ ID NO: 1 and has the amino acid sequence shown in SEQ ID) No: 2. Homologous genes with GDP-mannose-dehydratase activity include the porcine GMD, (Broschat et al., Eur J. Biochem., 153(2):397-401 (1995)), *Caenorhabditis elegans* GMD and *Drosophila melanogaster GMD* (*See,* for example, Rhomberg et al., FEBS J.; 273:2244-56 (2006)); *Arabitopsis thaliana;* (*See,* e.g, Nakayama et al.,Glycobiology; 13:673-80 (2003)); and *E. coli*, (Somoza et al.,Structure,8:123-35 (2000)).

GDP-keto-deoxy-mannose-epimerase/GDP-keto-deoxy-galactose-reductase (GDP-L-fucose synthase, EC1.1.1. 271) is a bifunctional enzyme, which has been identified in both eukaryotes and prokaryotes. The human GDP-keto-deoxy-mannose"epimerase/GDP:'keto-' deoxy-galactose-reductase is called the FX protein (also known as hFX or GER). The nucleotide sequence encoding the hFX is shown in SEQ:ID:NO:3. The hRX protein has the amino acid sequence shown in SEQ ID NO: 4:

The GDP-fucose transporter has been identified in several species. The human GDP-fucose transporter (hGFTr) has been identified as related to congenital disorders of glycosylation-II(CDG-II)(Lubke et al., Nat. Genet. 28:73-6 Also known as Leukocyte Adhesion Deficiency II(LAD II), it appears that the disorder results from a disturbance in fucosylation of selectin ligands. Roos and Law; Blood Cells Mol.Dis.27: 1000-4-(2001). The nucleotide sequence encoding the hGFTr is shown in SEQ ID NO: 5 and the amino acid sequence of the hGFTr is shown in SEQ ID NO: 6. Homologous genes with GDP-fucose transporter activity have been identified in other species, such as *Drosophila melanogaster* (Ishikawa et al., Proc. Natl. Acad. Sci. U S A. 102:18532-7 (2005)), rat liver (Puglielli and Hirschberg; J. Biol. Chem. 274:35596-60 (1999)), and a putative CHO homolog (Chen et al., Glycobiology;15:259-69 (2005)).

A number of fucosyltransferases have been identified (See Breton et al., Glycobiol. 8: 87-94 (1997); Becker, Lowe, Glycobiol. 13: 41R-53R (2003); Ma et al., Glycobiol. 16: 158R-184R (2006)), for example, α1,2-fucosyltransferase (EC 2.4.1.69; encoded by *FUT1* and *FUT2*), α1,3-fucosyltransferase (glycoprotein 3-α-L-fucosyltransferase, EC 2.4.1.214; encoded by *FUT3-FUT7* and *FUT9*), α1,4-fucosyltransferase (EC 2.4.1.65; encoded by *FUT3*), and α1,6-fucosyltransferase (glycoprotein 6-α-L-fucosyltransferase, EC 2.4.1.68; encoded by *FUT8*). In general, α1,2-fucosyltransferase transfer fucose to the terminal galactose residue in an *N*-glycan by way of an α1,2 linkage. In general, the α1,3-fucosyltra.nsferase and α1,4-fucosyltransferases transfer fucose to a GlcNAc residue at the non-reducing end of the *N-*glycan.

In general, α1,6-fucosyltransferases transfer fucose by way of an α1,6-linkage to the GlcNAc residue at the reducing end of *N*-glycans (asparagine-linked GlcNAc). Typically, α1,6-fucosyltransferase requires a terminal GlcNAc residue at the non-reducing end of at least one branch of the trimannose core to be able to add fucose to the GlcNAc at the reducing end. However, an α1,6-fucosyltransferase has been identified that requires a terminal galactoside residue at the non-reducing end to be able add fucose to the GlcNAc at the reducing end (Wilson et al., Biochm. Biophys. Res. Comm. 72: 909-916 (1976)) and Lin et al. (Glycobiol. 4: 895-901 (1994)) has shown that in Chinese hamster ovary cells deficient for GlcNAc transferase I, the α1,6-fucosyltansferase will fucosylate Man₄GlcNAc₂ and Man₅GlcNAc₂ *N*-glycans. Similarly, α1,3-fucosyltransferase transfers to the GlcNAc residue at the reducing end of *N*-glycans but by way of an α1,3-linkage, generally with a specificity for *N*-glycans with one unsubstituted non-reducing terminal GlcNAc residue. The *N*-glycan products of this enzyme are present in plants, insects, and some other invertebrates (for example, *Schistosoma, Haemonchus, Lymnaea).* However, U.S. Patent No. 7,094,530 describes an α1,3-fucosyltransferase isolated from human monocytic cell line THP-1.

The human α1,6-fucosyltransferase (*hFUT8*) has been identified by Yamaguchi et al., (Cytogenet. Cell. Genet. 84: 58-6 (1999)). The nucleotide sequence encoding the human *FUT8* is shown in SEQ ID NO:7. The amino acid sequence of the *hFUT8* is shown in SEQ ID NO: 8. Homologous genes with *FUT8* activity have been identified in other species, such as a rat *FUT8 (rFUT8)* having the amino acid sequence shown in SEQ ID NO: 10 and encoded by the nucleotide sequence shown in SEQ ID NO: 9; a mouse Fut8 (m*FUT8*) having the amino acid sequence shown in SEQ ID NO: 12 and encoded by the nucleotide sequence shown in SEQ ID NO:11, and a porcine *FUT8* (*pFUT8*) having amino acid sequence shown in SEQ ID NO: 14 and encoded by the nucleotide sequence shown in SEQ ID NO: 13. *FUT8* has also been identified in CHO cells (Yamane-Ohnuki et al., Biotechnol. Bioeng. 87: 614-622 (2004)), monkey kidney COS cells (Clarke and Watkins, Glycobiol. 9: 191-202 (1999)), and chicken cells (Coullin et al., Cytogenet. Genome Res. 7: 234-238 (2002)). Paschinger et al., Glycobiol. 15: 463-474 (2005) describes the cloning and characterization of fucosyltransferases from *C*. *elegans* and *D. melanogaster. Ciona intestinalis, Drosophila pseudoobscura, Xenopus laevis,* and *Danio rerio* putative α1,6-fucosyltransferases have been identified (GenBank accession numbers AJ515151, AJ830720, AJ514872, and AJ781407, respectively).

The aforementioned fucosylation pathway enzymes or activities are encoded by nucleic acids. The nucleic acids can be DNA or RNA, but typically the nucleic acids are DNA because it preferable that the nucleic acids encoding the fucosylation pathway enzymes or activities are stably integrated into the genome of the host cells. The nucleic acids encoding the fucosylation pathway enzymes or activities are each operably linked to regulatory sequences that allow expression of the fucosylation pathway enzymes or activities. Such regulatory sequences include a promoter and optionally an enhancer upstream of the nucleic acid encoding the fucosylation pathway enzyme or activity and a transcription termination site downstream of the fucosylation pathway enzyme or activity. The nucleic acid also typically further includes a 5' untranslated region having a ribosome binding site and a 3' untranslated region having a polyadenylation site. The nucleic acid is often a component of a vector such as a plasmid, which is replicable in cells in which the fucosylation pathway enzyme or activity is expressed. The vector can also contain a marker to allow selection of cells transformed with the vector. However, some cell types, in particular yeast, can be successfully transformed with a nucleic acid that lacks vector sequences.

In general, the host cells transformed with the nucleic acids encoding the one or more fucosylation pathway enzymes or activities further includes one or more nucleic acids encoding desired glycoproteins. Like for the fucosylation pathway enzymes, the nucleic acids encoding the glycoproteins are operably linked to regulatory sequences that allow expression of the glycoproteins. The nucleic acids encoding the glycoproteins can be amplified from cell lines known to express the glycoprotein using primers to conserved regions of the glycoprotein (*See,* for example, Marks et al., J. Mol. Biol. : 581-596 (1991)). Nucleic acids can also be synthesized *de novo* based on sequences in the scientific literature. Nucleic acids can also be synthesized by extension of overlapping oligonucleotides spanning a desired sequence (*See,* for example, Caldas et al., Protein Engineering, 13: 353-360 (2000)).

The type of fucosylated N-glycan structure produced by the host cell will depend on the glycosylation pathway in the host cell and the particular fucosyltransferase. For example, α1,2-fucosyltransferases in general add a fucose to the terminal galactose on an N-glycan. As such, a pathway that utilizes an α1,2-fucosyltransferase would preferably be introduced into a host cell that is capable of producing N-glycans having a Gal₂GlcNAc₂Man₃GlcNAc₂ glycoform. The N-glycans produced will have fucose in an α1,2 linkage to the terminal galactose residues. Both α1,3-fucosyltransferases and α1,4-fucosyltransferases add fucose to one or more GlcNAc residues at or near the non-reducing end by way of an α1,3 or α1,4 linkage, respectively, or for some α1,3-fucosyltransferases, by way of an α1,3 linkage to the core GlcNAc linked to the asparagine residue of the glycoprotein. As such, a pathway that utilizes an α1,3/4-fucosyltransferase would preferably be introduced into a host cell that is capable of producing N-glycans having at least a GlcNAcMan₅GlcNAc₂ glycoform. Finally, α1,6-fucosyltransferases in general transfer fucose by way of an α1,6 linkage to the core GlcNAc linked to the asparagine residue of the glycoprotein. In general, a pathway that utilizes an α1,6-fucosy\trarisferase would preferably be introduced into a host cell that is capable of producing N-glycans having at least a GlcNAcMan₅GlcNAc₂, Man₅GlcNAc₂, or Man₄GlcNAc₂ glycoform.

The glycoproteins that can be produced in accordance using the methods disclosed herein include any desired protein for therapeutic or diagnostic purposes, regardless of the origin of the nucleic acid sequence for producing the glycoprotein. For example, monoclonal antibodies in which the *N*-glycan is not fucosylated have increased ADCC activity; however, increased ADCC activity is undesirable for monoclonal antibodies intended to bind receptor ligands as a treatment for a disorder but not elicit ADCC activity. Monoclonal antibodies produced in the host cells disclosed herein comprising the fucosylation pathway will have fucosylated *N*-glycans and will be expected to have decreased ADCC activity. As another example, immunoadhesions (*See*, U.S. Patent Nos. 5,428,130, 5,116,964, 5,514,582, and 5,455,165; Capon et al. Nature 337:525 (1989); Chamow and Ashkenazi, Trends Biotechnol. 14: 52-60 (1996); Ashkenazi and Chamow, Curr. Opin. Immunol. 9: 195-200 (1997)), which comprise the extracellular portion of a membrane-bound receptor fused to the Fc portion of an antibody produced in the host cells disclosed herein comprising the fucosylation pathway will have fucosylated *N*-glycans and will be expected to have decreased ADCC activity. Examples of glycoproteins that can be produced according to the methods herein to have fucosylated *N-*glycans include, but are not limited to, erythropoietin (EPO); cytokines such as interferon-α, interferon-β, interferon-y, interferon-ω, and granulocyte-CSF; coagulation factors such as factor VIII, factor IX, and human protein C; monoclonal antibodies, soluble IgE receptor α-chain, IgG, IgM, IgG, urokinase, chymase, and urea trypsin inhibitor, IGF-binding protein, epidermal growth factor, growth hormone-releasing factor, annexin V fusion protein, angiostatin, vascular endothelial growth factor-2, myeloid progenitor inhibitory factor-1, osteoprotegerin tissue, plasminogen activator, G-CSF, GM-CSF, and TNF-receptor.

The nucleic acides fusion proteins comprising the catalytic domain of a fucosylation pathway protein fused to a targeting peptide, which targets the fusion protein to a particular region within the cell. The targeting peptide targets the fusion protein to a location within the secretory pathway. The term "secretory pathway" thus refers to organelles and components within the cell where glycoproteins are modified in preparation for secretion. The secretory pathway includes the endoplasmic reticulum (ER), the Golgi apparatus, the trans-Golgi network, and the secretory vesicles. For example, suitable cellular targeting peptides may target the catalytic domain to the ER, the Golgi apparatus, the trans-Golgi network, or secretory vesicles. Targeting peptides which may be useful in the present invention include those described in US. Patent No. 7,029 872. In one embodiment, the catalytic domain of the fucosyltransferase is fused to a targeting peptide mat directs the fusion protein to the Gogi apparatus. The particular targeting peptide fused to the fucosyltransferase catalytic domain will depend on the host cell the particular fucosyl transferase and, the glycoprotein produced. Examples of targeting peptides that can be used for targeting the fucosyltransferase have been disclosed in, for example, U.S. Patent No. 7,029,872 and U.S. Published Patent Application Nos. 200470018590, 2004/0230042, 2005/0248617, 2004/0171826, 2006/0286637, and 2007/0037248.

The nucleic acids encoding the enzymes or activities involved in the fucosylation pathway are ligated into vectors, which are capable of being used to transfect host cells. Typically, the vectors will include regulatory elements, which have been isolated from the same species of cell as the intended host cell, or which have been isolated from other species, but which are known to be functional when inserted into the intended host cell. Typically, these regulatory elements include 5' regulatory sequences, such as promoters, as well as 3' regulatory sequences, such as transcription terminator sequences. Vectors will typically also include at least one selectable marker element that allows for selection of host cells that have been successfully transfected with the vector. The vectors are transferred into the intended host cells, and the resulting cells are screened for the presence of the selectable marker, to identity those hostcells which have been successfully transfected with the vector, and which will therefore also carry the vector encoding the fusion protein.

P.pastoris can be genetically modified so that they express glycoproteins, in which the glycosylation pattern is complex or human-like or humanized. Such genetically modified lower eukaryotes can be achieved by eliminating selected endogenous glycosylation enzymes that are involved in producing high mannose *N*-glycans and introducing various combinations of exogenous enzymes involved in making complex *N*-glycans. Methods for genetically engineering yeast to produce complex *N*-glycans has been described in U.S. Patent No. 7,029,872 and U.S. Published patent Application Nos. 2004/0018590, 2005/0170452, 2006/0286637, 2004/0230042, 2005/0208617, 2004/0171826, 2005/0208617, and 2006/0160179. For example, a host cell is selected or engineered to be depleted in 1,6₌mannosyl transferase activities, which would otherwise add mannose residues on to the N-glycan on a glycoprotein. For example, in yeast, the *OCH1* gene encodes 1,6-mannosyl transferase activity. The host cells is then further engineered include one or more of the enzymes involved in producing complex, human-like *N*-glycans.

In one embodiment, the host cell further includes in α1,2-manosidase catalytic domain fused to a cellular targeting signal peptide not normally associated with the catalytic domain and selected to target the α1,2-mannosidase activity to the ER or Golgi apparatus of the host cell. Passage of a recombinant, glycoprotein through the ER or Golgi apparatus of the host cell produces a recombinant glycoprotein comprising a fucosylated Man₅GlcNAc₂ glycoform, for example a Man₅GlcNAc₂(Fuc) glycoform. U.S. Patent No, 7,029,872 and U.S. Published Patent Application Nos. 2004/0018590 and 2005/0 170452 disclose lower eukaryote host cells capable of producing a glycoprotein comprising a Man₅GlcNAc₂ glycoform.

In a further embodiment the immediately preceding host cell further includes a GlcNAc transferase I (GnTI) catalytic domain fused to a cellular targeting signal peptide not normally associated with the catalytic domain and selected to target GlcNAc transferase I activity to the ER or Golgi apparatus of the host cell. Passage of the recombinant glycoprotein through the ER or Golgi apparatus of the host cell produces a recombinant glycoprotein comprising a fucosylaled GlcNAcMan₅GlcNAc₂ glycoform, for example a GlcNAcMan₅GlcNAc₂(Fuc) glycoform. U.S. Patent No, 7,029,872 and U.S. Published Patient Application Nos. 2004/0018590 and 2005/0176452 disclose lower eukaryote host cells capable of producing a glycoprotein comprising a GlcNAcMan₃GlcNAc₂ glycoform. The glycoprotein produced in the above cells can be treated *in vitro* with a hexaminidase to produce a recombinant glycoprotein comprising a fucosylated Man₅GlcNAc₂(Fuc) glycoform.

In a further still embodiment, the immediately preceding host cell further includes a mannosidase II catalytic domain fused to a cellular targeting signal peptide not normally associated with the catalytic domain and selected to target mannosidase II activity to the ER or Golgi apparatus of the host cell. Passage of the recombinant glycoprotein through the ER or Golgi apparatus of the host cell produces a recombinant glycoprotein comprising a fucosylated GlcNAcMan₃GlcNAc₂ glycoform, for example a GlcNAcMan₃GlcNAc₂(Fuc) glycoform. U.S. Published Patent Application No. 2004/0230042 discloses lower eukaryote host cells that express mannosidase II enzymes and are capable of producing glycoproteins having predominantly a GlcNAc₂Man₃GlcNAc₂ glycoform. The glycoprotein produced in the above cells can be treated *in vitro* with a hexaminidase to produce a recombinant glycoprotein comprising a Man₃GlcNAc₂(Fuc) glycoform.

In a further still embodiment, the immediately preceding host cell further includes GlcNAc transferase II (GnTII) catalytic domain fused to a cellular targeting signal peptide not normally associated with the catalytic domain and selected to target GlcNAc transferase II activity to the ER or Golgi apparatus of the host cell. Passage of the recombinant glycoprotein through the ER or Golgi apparatus of the host cell produces a recombinant glycoprotein comprising a fucosylated GlcNAc₂Man₃GlcNAc₂ glycoform, for example a GlcNAc₂Man₃GlcNAc₂(Fuc) glycoform. U.S. Patent No, 7,029,872 and U.S. Published Patent Application Nos. 2004/0018590 and 2005/0170452 disclose lower eukaryote host cells capable of producing a glycoprotein comprising a GlcNAc₂Man₃GlcNAc₂ glycoform. The glycoprotein produced in the above cells can be treated *in vitro* with a hexaminidase to produce a recombinant glycoprotein comprising a Man₃GlcNAc₂(Fuc) glycoform.

In a further still embodiment, the immediately preceding host cell further includes a Galactose transferase II catalytic domain fused to a cellular targeting signal peptide not normally associated with the catalytic domain and selected to target Galactose transferase II activity to the ER or Golgi apparatus of the host cell. Passage of the recombinant glycoprotein through the ER or Golgi apparatus of the host cell produces a recombinant glycoprotein comprising a fucosylated Gal₂GlcNAc₂Man₃GlcNAc₂ glycoform, for example a Gal₂GlcNAc₂Man₃GlcNAc₂(Fuc) glycoform. U.S. Published Patent Application No. 2006/0040353 discloses lower eukaryote host cells capable of producing a glycoprotein comprising a Gal₂GlcNAc₂Man₃GlcNAc₂ glycoform. The glycoprotein produced in the above cells can be treated *in vitro* with a galactosidase to produce a recombinant glycoprotein comprising a fucosylated GlcNAc₂Man₃GlcNAc₂ glycoform, for example a GlcNAc₂Man₃GlcNAc₂(Fuc) glycoform.

In a further still embodiment, the immediately preceding host cell further includes a sialytransferase catalytic domain fused to a cellular targeting signal peptide not normally associated with the catalytic domain and selected to target sialytransferase activity to the ER or Golgi apparatus of the host cell. Passage of the recombinant glycoprotein through the ER or Golgi apparatus of the host cell produces a recombinant glycoprotein comprising a fucosylated NANA₂Gal₂GlcNAc₂Man₃GlcNAc₂ glycoform, for example, a NANA₂Gal₂GlcNAc₂Man₃GlcNAc₂(Fuc)glycoform. For lower eukaryote host cells such as yeast and filamentous fungi, it is preferred that the host cell further include a means for providing CMP-sialic acid for transfer to *N*-glycan. Published Patent Application No. 2005/0260729 discloses a method for genetically engineering lower eukaryotes to have a CMP-sialic acid synthesis pathway and UTS published Patent Application No. 2006/0286637 discloses a method for genetically engineering lower eukaryotes to produce sialylated glycoproteins. The glycoprotein produced in the above cells can be treated *in vitro* with a heuraminidase to produce a recombinant glycoprotein comprising a fucosylated Gal₂GkcNAc₂Man₃GlcNAc₂ glycoform, for example, a Gal₂GlcNAc₂Man₃GlcNAc₂(Fuc) glycoform.

Any one of the preceding host cells can further include one or more GlcNAc transferase selected from the group consisting of GnTIII, GnTIV, GnTV, GnTVI, and GnTIX to produce glycoprotens having bisected and/or multiantennary *N*-glycan structures such as disclosed in U.S. Published Patent Application Nos. 2004/074458 and 2007/0037248. Various of the preceding host cells further include one or more sugar transporters such as UDP-GlcNAc. transporters (for example, *Kluyveromyces lactis and Mus musculus* UDP-GlcNAc transporters), UDP-galactose transporters (for example, *Drosophila melanogaster* UDP-galactose transporter), and CMP-sialic acid transporter (for example, human static acid transporter). Because P. pastoris lacks the above transporters, it is preferable that it be genetically engineered to include the above transporters,

In further embodiments of the above host cells, the host cells are further genetically engineered to eliminate glycoproteins having α-mannosidase-resistant N-glycans by deleting or disrupting the β-mannosyltransferase gene (*BMT2*)(*See* ,U.S. Published Patent Application No. 2006/0211085) and glycoproteins having phosphomannose residues by deleting or disrupting one or both of the phosphomannosyl transferase genes *PNO1* and *MNN4B* (*See* for example, U.S. Published Patent Application Nos. 2006/0160179 and 2004/0014170). In further still embodiments of the above host cells, the host cells are further genetically modified to eliminate O-glycosylation of the glycoprotein by deleting or disrupting one or more of the Dol-P-Man:Protein (Ser/Thr) Mannosyl Transferase genes (*PMTs*) (See U.S. Patent No. 5,714,377).

It has been shown that codon optimization of genes or transcription units coding for particular polypeptides leads to increased expression of the encoded polypeptide, that is increased translation of the mRNA encoding the polypeptide. Therefore, in the case of the host: cells disclosed herein, increased expression of the encoded enzymes will produce more of the encoded enzymes, which can lead to increased production of *N*-glycans that are fucosylated. In the context of codon optimization, the term "expression" and its variants refer to translation of the mRNA encoding the polypeptide and not to transcription of the polynucleotide encoding the polypeptide. The term "gene" as used herein refers to both the genomic DNA or RNA encoding a polypeptide and to the cDNA encoding the polypeptide.

Codon optimization is a process that seeks to improve heterologous expression of a gene when that gene is moved into a foreign genetic environment that exhibits a different nucleotide codon usage from the gene's native genetic environment or improve ectopic expression of a gene in its native genetic environment when the gene naturally includes one or more nucleotide codons that are not usually used in genes native to the genetic environment that encode highly expressed genes. In other words, codon optimization involves replacing those nucleotide codons of a gene that are used at a relatively low frequency in a particular genetic environment or organism with nucleotide codons that are used in genes that are expressed at a higher frequency in the genetic environment or organism. In that way, the expression (translation) of the gene product (polypeptide) is increased. The assumption is that the nucleotide codons that appear with high frequency in highly expressed genes are more efficiently translated than nucleotide codons that appear at low frequency.

In general, methods for optimizing nucleotide codons for a particular gene depend on identifying the frequency of the nucleotide codons for each of the amino acids used in genes that are highly expressed in an organism and then replacing those nucleotide codons in a gene of interest that are used with low frequency in the highly expressed genes with nucleotide codons that are identified as being used in the highly expressed genes (*See* for example Lathe, Synthetic Oligonucleotide Probes Deduced from Amino Acid Sequence Data: Theoretical and Practical Considerations, J. Molec. Biol.: 183: 1-12 (1985); Nakamura et al., Nuc. Acid Res. 28: 292 (2000); Fuglsang, Protein Expression & Purification 31: 247-249 (2003)). There are numerous computer programs that will automatically analyze the nucleotide codons of a nucleic acid of an organism encoding a gene and suggest nucleotide codons to replace nucleotide codons, which occur with low frequency in the organism, with nucleotide codons that are found in genes that are highly expressed in the organism.

The following examples are intended to promote a further understanding of the present invention.

### EXAMPLE 1

This Example shows the construction of a *Pichia pastoris* strain capable of producing glycoproteins that include fucose in the N-glycan structure of the glycoprotein.

*Escherichia coli* strains TOP10 or XL10-Gold are used for recombinant DNA work. PNGase-F, restriction and modification enzymes are obtained from New England BioLabs (Beverly, MA), and used as directed by the manufacturer. α-1,6-Fucosidase is obtained from Sigma-Aldrich (St. Louis, MO) and used as recommended by the manufacturer. Oligonucleotides are obtained from Integrated DNA Technologies (Coralville, IA). Metal chelating "HisBind" resin is obtained from Novagen (Madison, WI). 96-well lysate-clearing plates are from Promega (Madison, WI). Protein-binding 96-well plates are from Millipore (Bedford, MA). Salts and buffering agents are from Sigma-Aldrich (St. Louis, MO).

### Amplification of fucosylation pathway genes.

An overview of the fucosylation pathway is shown in Figure 1. The open reading frame (ORF) of hGMD is amplified from human liver cDNA (BD Biosciences, Palo Alto, CA) using Advantage 2 polymerase following the procedure recommended by the manufacturer. Briefly, the primers SH415 and SH413 (5'- GGCGG CCGCC ACCAT GGCAC ACGCA CCGGC ACGCT GC-3' (SEQ ID NO: 15) and 5'- TTAAT TAATC AGGCA TTGGG GTTTG TCCTC ATG-3' (SEQ ID NO: 16), respectively) are used to amplify a 1,139 bp product from human liver cDNA using the following conditions: 97°C for 3 minutes; 35 cycles of 97°C for 30 seconds, 50°C for 30 seconds, 72°C for 2 minutes; and 72°C for 10 minutes. Subsequently the product is cloned into pCR2.1 (Invitrogen, Carlsbad, CA), sequenced, and the resultant construct designated pSH985.

Using the conditions outlined above, the primers SH414 and SH411 (5'-GGCGG CCGCC ACCAT GGGTG AACCC CAGGG ATCCA TG-3' (SEQ ID NO: 17) and 5'-TTAAT TAATC ACTTC CGGGC CTGCT CGTAG TTG-3' (SEQ ID NO:18), respectively) are used to amplify a 986 bp fragment from human kidney cDNA (BD Biosciences, Palo Alto, CA), which corresponds to the ORF of the human FX gene. Subsequently, this fragment is cloned into pCR2.1, sequenced, and designated pSH988.

The ORF of the human GFTr is amplified from human spleen cDNA (BD Biosciences, Palo Alto, CA) using the conditions outlined above, and the primers RCD679 and RCD680 (5'-GCGGC CGCCA CCATG AATAG GGCCC CTCTG AAGCG G-3' (SEQ ID NO: 19) and 5'-TTAAT TAATC ACACC CCCAT GGCGC TCTTC TC-3' (SEQ ID NO:20), respectively). The resultant 1,113 bp fragment is cloned into pCR2.1, sequenced, and designated pGLY2133.

A truncated form of the mouse *FUT8* ORF, encoding amino acids 32 to 575 and lacking the nucleotides encoding the endogenous transmembrane domain, is amplified from mouse brain cDNA (BD Biosciences, Palo Alto, CA) using the conditions outlined above and the primers SH420 and SH421 (5'- GCGGC GCGCC GATAA TGACC ACCCT GATCA CTCCA G-3' (SEQ ID NO:21) and 5'- CCTTA ATTAA CTATT TTTCA GCTTC AGGAT ATGTG GG-3' (SEQ ID NO:22), respectively). The resultant 1,654 bp fragment is cloned into pCR2.1, sequenced, and designated pSH987.

### Generation of fucosylation genes in yeast expression cassettes.

Open reading frames for GMD, FX, and GFTr are generated by digesting the above vectors with *Not*I and *Pac*I restriction enzymes to produce DNA fragments with a *Not*I compatible 5' end and a *Pac*I compatible 3' end. The FUT8 fragment is generated by digesting with *Asc*I and *Pac*I restriction enzymes to produce a DNA with an *Asc*I compatible 5' end and a *Pac*I compatible 3' end.

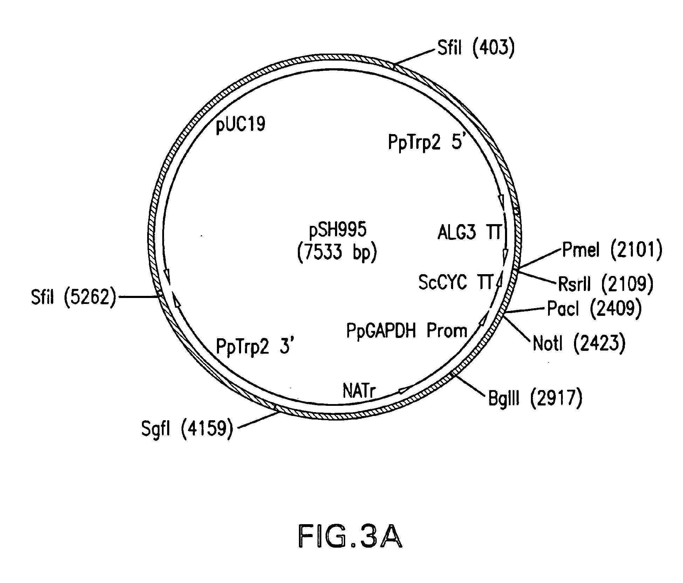

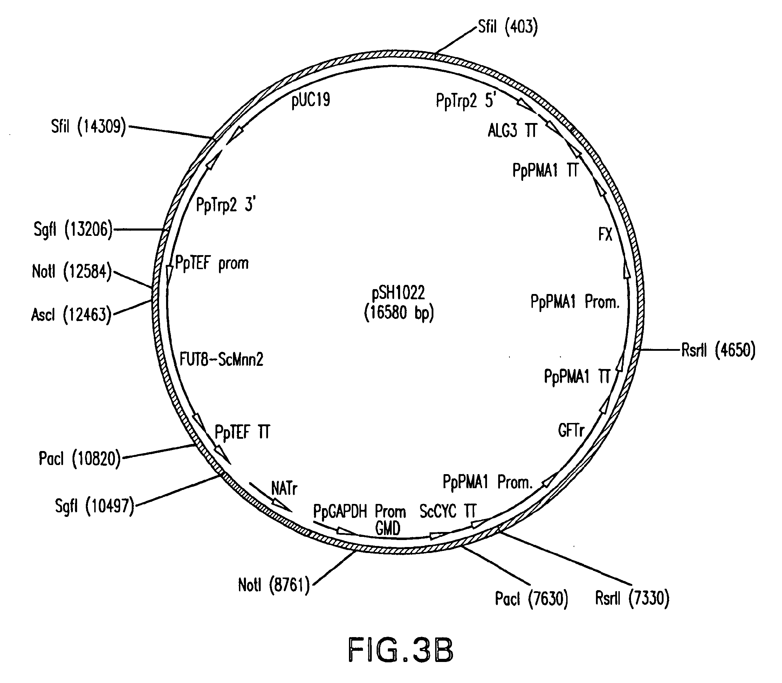

To generate the GMD expression cassette, GMD is cloned into yeast expression vector pSH995, which contains a *P. pastoris* GAPDH promoter and *S*. *cerevisiae* CYC transcription terminator sequence and is designed to integrate into the *Pichia* genome downstream of the *Trp2* ORF, using the nourseothricin resistance marker. This vector is illustrated in Figure 3A. The vector pSH985 is digested with *Not*I and *Pac*I to excise a 1.1 Kb fragment containing the GMD ORF, which is then subcloned into pSH995 previously digested with the same enzymes. The resultant vector, containing GMD under the control of the GAPDH promoter, is designated pSH997. A.

To generate the FX expression cassette, the vector pSH988 is digested with *Not*I and *Pac*I to excise a 1.0 Kb fragment containing the FX ORF, which is treated with T4 DNA polymerase to remove single strand overhangs (J. Sambrook, D. W. Russell, Molecular Cloning: A laboratory Manual (Cold Spring Harbor Laboratory Press, Cold Spring. Harbor, NY, ed. 3rd, 2001)). Subsequently this fragment is subcloned into the vector pGLY359 (Hamilton et al., Science 313, 1441 (2006)) previously digested with *Not*I and *Asc*I, and treated with T4 DNA polymerase. The resultant vector, pSH994, contains an FX expression cassette consisting of the FX ORF operably linked at the 5' end to a *P. pastoris* PMA 1 promoter (PpPMA 1 prom) and at the 3' end to a *P. pastoris* PMA transcription terminator sequence (PpPMA1tt). The expression cassette is flanked by *Swa*I restriction sites.

The GFTr expression cassette is generated by digesting pGLY2133 with *Not*I and *Pac*I to excise a 1.1 Kb fragment containing the GFTr ORF, which is treated with T4 DNA polymerase. Subsequently this fragment is subcloned into the vector pGLY363 (Hamilton, *supra*), previously digested with *Not*I and *Pac*I, and treated with T4 DNA polymerase. The resultant vector, pGLY2143, contains a GFTr expression cassette consisting of the GFTr ORF operably linked at the 5' end to a PpPMA1 prom and at the 3' end to a PpPMA1tt. The expression cassette is flanked by *Rsr*II restriction sites.

To generate the FUT8 catalytic domain fused to a yeast localization signal, the first 36 amino acids of *S*. *cerevisiae* targeting region of *Mnn2* are analyzed by the GeneOptimizer software and codon-optimized for *P. pastoris* expression (GeneArt, Regensburg, Germany). The resultant synthetic DNA for *ScMnn2* amino acids 1 to 36 is generated with 5' *Not*I and 3' *Asc*I restriction enzyme compatible ends, cloned into a shuttle vector to produce plasmid vector pSH831. Subsequently, the vector pSH987 is digested with *Asc*I and *Pac*I to liberate a 1.6 Kb fragment encoding the FUT8 catalytic domain ORF, which is then subcloned in-frame to the DNA encoding the *ScMnn2* targeting peptide in the vector pSH831, previously digested with the same enzymes. The resultant vector is designated pSH989. To generate the FUT8-ScMnn2 expression cassette, pSH989 is digested with *Not*I and *Pac*I to release a 1.8 Kb fragment, which is subcloned into the vector pGLY361 (Hamilton et al., Science 313, 1441 (2006)) digested with the same enzymes. The resultant vector, pSH991, contains a FUT8-Mnn2 fusion protein consisting of the FUT8-Mnn2 fusion ORF operably linked at the 5' end to a *P. pastoris* TEF promoter (PpTEFprom) and at the 3' end to a *P. pastoris* TEF transcription terminator sequence (PpTEFtt). The expression cassette is flanked by *Sgf*I restriction sites.

### Generation of fucosylation engineering vector.

Vector pSH994 is digested with *Swa*I to release a 2.5 Kb fragment containing the FX expression cassette, which is subcloned into pSH997 (contains the GMD expression cassette) digested with *Pme*I. The resultant vector in which the PMA-FX and GAPDH-GMD expression cassettes are aligned in the same direction is designated pSH1009. The 2.7 Kb fragment containing the PMA-GFTr expression cassette is excised from pGLY2143 using the restriction enzyme *RsrI*I and subcloned into pSH1009 digested with the same enzyme. The resultant vector in which the PMA-GFTr and GAPDH expression cassettes are aligned in the same direction is designated pSH1019. Finally, the 1.8 Kb TEF-FUT8 cassette is excised from pSH991 using *Sgf*I and subcloned into pSH1019 digested with the same enzyme. The resultant vector in which the TEF-FUT8 and the GAPDH expression cassettes are aligned in the same direction is designated pSH1022. This vector is illustrated in Figure 2B.

### Generation of rat EPO expression vector.

A truncated form of *Rattus norvegicus* erythropoietin gene (rEPO), encoding amino acids 27 to 192, is amplified from rat kidney cDNA (BD Biosciences, Palo Alto, CA) using Advantage 2 polymerase as recommended by the manufacturer. Briefly, the primers rEPO-forward and rEPO-reverse (5'-GGGAA TTCGC TCCCC CACGC CTCAT TTGCG AC-3' (SEQ ID NO:23) and 5'-CCTCT AGATC ACCTG TCCCC TCTCC TGCAG GC-3' (SEQ ID NO:24), respectively) are used to amplify a 516 bp product from rat kidney cDNA using the following cycling conditions: 1 cycle at 94°C for 1 minute; 5 cycles at 94°C for 30 seconds, 72°C for 1 minute; 5 cycles at 94°C for 30 seconds, 70°C for 1 minute; 25 cycles at 94°C for 20 seconds, 68°C for 1 minute. Subsequently, the product is cloned into pCR2.1 (Invitrogen, Carlsbad, CA), sequenced, and the resultant construct designated pSH603. To generate the yeast expression vector, pSH603 is digested with *Eco*RI and *Xba*I to liberate a 506 bp fragment which was subcloned into pPICZαA (Invitrogen, Carlsbad, CA), which has previously been digested with the same enzymes. The resultant expression vector is designated pSH692. The rEPO in pSH692 is under the under the control of the AOX methanol-inducible promoter.

### Generation of yeast strains and production of rat EPO.

A *P. pastoris* glycoengineered cell line, YGLY1062, which is capable of producing recombinant glycoproteins having predominantly Gal₂GlcNAc₂Man₃GlcNAC₂ *N-*glycans (similar to the strains described in U.S. Published Patent Application No. 2006/0040353, which produce glycoproteins having Gal₂GleNAc₂Man₃GlcNAc₂ N-glycans) is transformed with vector PSH692 to produce strain RDP974, which produces recombinant rat EPO (rEPO) with Gal₂GlcNAc₂Man₃GlcNAc₂ N-glycans. Strain RDP974 is similar to stain RDP762 described in Hamilton et al., Science 313, 1441-1443 (2006), which produces rat EPO having Gal₂GlcNAc₂Man₃GlcNAc₂ N-glycans.

The RPD974 strain has deletions in the *OCB1, PNO1, MNN4B,* and *BMT2* genes and includes DNA encoding the full-length *Kluyveromyces lactis* UDP-GlcNAc transporter, *M. musculus* UDP-GlcNAc transporter, *S*. *cerevisiae* UDP-galactose 4-epimerase, and *D*. *melanogaster* UDP-Galactose transporter; and DNA encoding a *M*. *musculus* α1,2-Mannosidase I catalytic domain fused to DNA encoding amino acids 1-36 of an *S*. *cerevisiae* MNN2 leader sequence; DNA encoding the *H. sapiens* β1,2-GlcNAc transferase I (GnTI) catalytic domain fused to DNA encoding amino acids 1-36 of an S. *cerevisiae* MNN2 leader sequence; DNA encoding a *Drosophila melanogaster* Mannosidase II catalytic domain fused to DNA encoding amino acids 1-36 of an *S*. *cerevisiae* MNN2 leader sequence, DNA encoding a *Rattus norvegicus* β1,2-GlcNAc transferase II (GnTII) catalytic domain fused to DNA encoding amino acids 1-97 of an S. *cerevisiae* MNN2 leader sequence, and DNA encoding an *H. sapiens* β1,4-galactosyltransferase (GalTI) catalytic domain fused to DNA encoding amino acids 1-58 of an S. *cerevisiae* KRE2 (MNTI) leader sequence. U.S. Published Patent Application No. 2006/0040353 discloses methods for producing *Pichia pastoris* cell lines that produce galactosylated glycoproteins in lower yeast (*See* also, U.S. Patent No. 7,029,872, U.S. Published Patent Application Nos. 2004/0018590, 2004/0230042, 2005/0208617, 2004/0171826, 2006/0286637, and 2007/0037248, and Hamilton et al., Science 313, 1441-1443 (2006).

Strain RDP974 is then used as the host strain for introducing the fucosylation pathway in vector pSH1022. Briefly, 10 µg of the control plasmid pSH995 or the fucosylation pathway plasmid pSH1022 is digested with the restriction enzyme *Sfi*I to linearize the vector and transformed by electroporation into the host strain RDP974. The transformed cells are plated on YPD containing 100 ng/mL nourseothricin and incubated at 26°C for five days. Subsequently several clones are picked and analyzed for fucose transfer onto the *N*-glycans of rEPO. A strain transformed with the control vector is designated YSH660, while a strain transformed with pSH1022 and demonstrating fucose transfer is designated YSH661.

Typically, protein expression is carried out by growing the transformed strains at 26°C in 50 mL buffered glycerol-complex medium (BMGY) consisting of 1% yeast extract, 2% peptone, 100 mM potassium phosphate buffer, pH 6.0, 1.34% yeast nitrogen base, 4 X 10-5% biotin, and 1 % glycerol as a growth medium. Induction of protein expression is performed in 5 mL of buffered methanol-complex medium (BMMY), consisting of 1.5% methanol instead of glycerol in BMGY.

Recombinant rEPO is expressed as described above and Ni-chelate column purified as described in Choi et al. (Proc. Natl. Acad. Sci. U S A 100, 5022 (2003) and Hamilton et al. (Science 301, 1244 (2003)). The resultant protein is analyzed by SDS-PAGE (Laemmli, Nature 227, 680 (1970)) and stained for visualization with coomassie blue. Fucose is removed by *in vitro* digestion with α-1,6-fucosidase (Sigma-Aldrich, St. Louis, MO) treatment, as recommended by the manufacturer.

For glycan analysis, the glycans are released from rEPO by treatment with PNGase-F (Choi et al. (2003); Hamilton *et al.* (2003)). Released glycans are analyzed by MALDI/Time-of flight (TOF) mass spectrometry to confirm glycan structures (Choi *et al.* (2003)). To quantitate the relative amount of fucosylated glycans present, the *N*-glycosidase F released glycans are labeled with 2-aminobenzidine (2-AB) and analyzed by HPLC (Choi *et al.* (2003)). The percentage of fucosylated and non-fucosylated glycans is calculated by comparing the peak area of each species before and after fucosidase treatment.

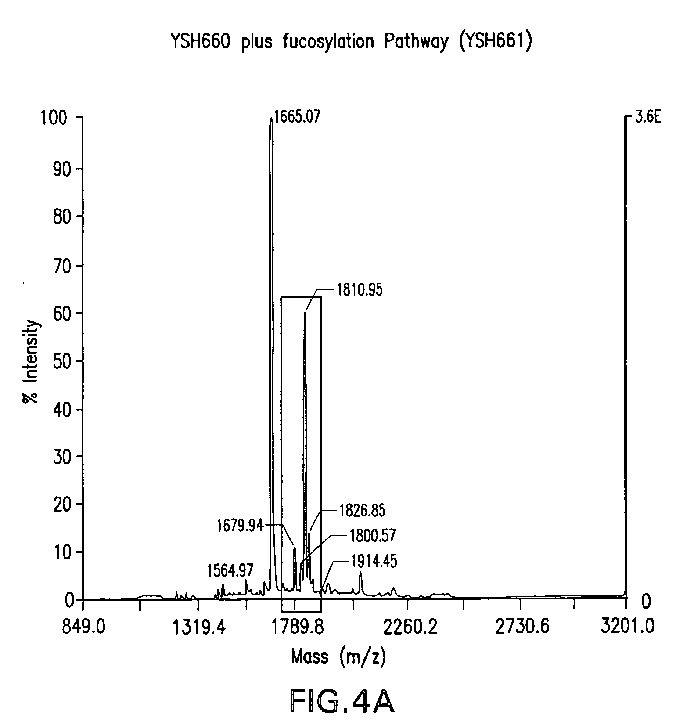

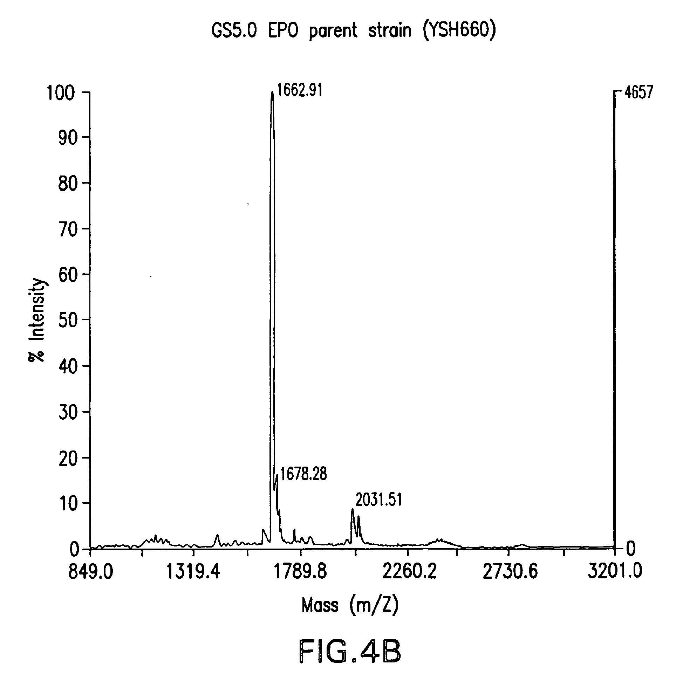

Analysis of the *N*-glycans produced in strain YSH661 produced essentially as described above showed that the strain produced recombinant rEPO comprising Gal₂GlcNAc₂Man₃GlcNAc₂(Fuc) *N*-glycans. Figure 4A, which shows the results of a MALDI-TOF analysis of the *N*-glycans on rEPO produced in strain YSH661, shows that the strain produced *N*-glycans comprised Gal₂GlcNAc₂Man₃GlcNAc₂(Fuc) Gal₂GlcNAc₂Man₃GlcNAc₂ *N*-glycans. The Gal₂GleNAc₂Man₃GlcNAc₂(Fuc) *N*-glycans are within the box. Figure 4B, which shows a MALDI-TOF analysis of the *N*-glycans on rEPO produced in control strain YSH660 (without fucosylation pathway), shows that the strain produced only afucosylated *N-*glycans comprising only Gal₂GlcNAc₂Man₃GlcNAc₂.

### EXAMPLE 2

A *Pichia pastoris* strain capable of producing glycoproteins having NANA₂Gal₂GlcNAc₂Man₃GlcNAc₂(Fuc) *N*-glycans can be made by introducing the vector pSH1022 into a *Pichia pastoris* strain capable of producing glycoproteins having NANA₂Gal₂GlcNAc₂Man₃GlcNAc₂ *N*-glycans. For example, vector pSH1022 containing the genes encoding the components of the fucosylation pathway can be transformed into the strain YSH597, which produces rat EPO having NANA₂Gal₂GlcNAc₂Man₃GlcNAc₂ N-glycans and is disclosed in U.S. Provisional Application No. 60/801,688 and Hamilton et al. Science 313, 1441-1443 (2006). The rat EPO produced in the strain upon induction will include NANA₂Gal₂GlcNAc₂Man₃GlcNAc₂(Fuc) *N*-glycans.

The following provides a prophetic method for introducing the enzymes encoding the sialylation pathway into strain YSH661 of Example 1.

Open reading frames for *Homo sapiens* UDP-N-acetylglucosamine-2-epimerase/N-acetylmannosamine kinase (GNE), *H sapiens* N-acetylneuraminate-9-phosphate synthase (SPS), *H*. *sapiens* CMP-sialic acid synthase (CSS), *Mus musculus* CMP-sialic acid transporter (CST), and amino acids 40 to 403 of *M. musculus* α-2,6-sialyltransferase (ST) are analyzed by the GeneOptimizer software and codon-optimized for *P. pastoris* expression (GeneArt, Regensburg, Germany). The resultant synthetic DNAs for GNE, SPS, CSS and CST are generated with 5' *Bsa*I and 3' *Hpa*I restriction sites, cloned into a shuttle vector and designated pGLY368, 367, 366 and 369, respectively. The synthetic DNA for ST is generated with 5' *Asc*I and 3' *PacI* restriction sites, cloned into a shuttle vector, and designated pSH660. To generate the SPS, CSS and CST expression cassettes, the vectors pGLY367, 366 and 369 are digested with *Bsa*I and *Hpa*I to excise 1.1, 1.3, and 1.0 Kb fragments, which are treated with T4 DNA polymerase to remove single strandoverhangs. Subsequently, these fragments are subcloned into the vectors pGLY359, 17, and 363 previously digested with *Not*I and *Asc*I for the former, and *NotI* and *Pac*I for the latter two, and treated with T4 DNA polymerase. The resultant vectors pSH819, containing SPS in a PpPMA1prom-PpPMA1tt cassette flanked by *Pac*I restriction sites; pSH824, containing CSS in a PpGAPDH-ScCYCtt cassette flanked by 5' *Bgl*II and 3' *Bam*HI restriction sites; and pGLY372, containing CST in a PpPMAlprom-PpPMA1tt cassette flanked by *RsrII* restriction sites. To generate the ST catalytic domain fused to a yeast localization signal, the S. *cerevisiae* targeting region of *Mnt1* is amplified from genomic DNA using Taq DNA polymerase (Promega, Madison, WI) and the primers ScMnt1-for and ScMnt1-rev (5'-GGGCGGCCGCCACCATGGCCCTC=CTC AGTAAGAGACT GTTGAG-3' (SEQ ID NO:25) and 5'-CCGGCGCGCCCGATGACTTGTTG TTCAGGGGATATAGATCCTG-3' (SEQ ID NO:26), respectively). The conditions used are: 94°C for 3 minutes, 1 cycle; 94°C for 30 seconds, 55°C for 20 seconds, 68°C for 1 minute, 30 cycles; 68°C for 5 minutes, 1 cycle. The resultant 174 bp fragment containing 5' *Not*I and 3' *Asc*I restriction sites is subcloned in-frame 5' to the codon-optimized ST, creating the vector pSH861. Subsequently this vector is digested with *NotI* and *PacI* to excise a 1.3Kb fragment, containing the ST-fusion, treated with T4 DNA polymerase and subcloned into pGLY361 prepared by digestion with *NotI* and *PacI,* and treated with T4 DNA polymerase. The resultant vector, containing the ST-fusion in a PpTEFprom-PpTEFtt cassette flanked by *Sgf*I restriction sites, is designated pSH893.

A yeast expression vector pSH823, containing a *P. pastoris* GAPDH promoter and S. *cerevisiae* CYC transcription terminator, is designed to integrate into the *Pichia* genome downstream of the *Trp2* ORF. The 2.6Kb fragment encoding the PMA-CST expression cassette is excised from pGLY372 using the restriction enzyme RsrII and subcloned into pSH823 digested with the same enzyme. The resultant vector in which the PMA-CST and GAPDH expression cassettes are aligned in the same direction was designated pSH826. Subsequently this vector is digested with the restriction enzymes *Not*I and *Pac*I and the single strand overhangs removed with T4 DNA polymerase. Into this linearized construct, the 2.2Kb fragment of GNE, isolated from pGLY368 by digestion with *Bsa*I *Hpa*I,and treated with T4 DNA polymerase to remove single strand overhangs, is subcloned. This vector is designated pSH828. Subsequently this vector is digested with *Pac*I, into which the 2.7 Kb *Pac*I fragment of pSH819, encoding the PMA-SPS expression cassette, is subcloned. The vector produced, in which the PMA-SPS expression cassette is aligned in the opposite orientation to the GAPDH expression cassette, is designated pSH830. At this stage the *URA3* marker is replaced with *HIS1* by excising the 2.4Kb U fragment from pSH830 using *Xho*I and replacing it with the the 1.8 Kb fragment of *HIS1* from pSH842 digested with the same enzyme. The resultant vector in which the *HIS1* ORF is aligned in the same direction as CAPDH-GNE expression cassette is designated pSH870. Subsequently this vector is digested with *Bam*HI and the 2.1 Kb fragment from pSH824 isolated by digestion with *Bdm*HI and *Bgl*II, containing the GAPDH-CSS expression cassette, is subcloned. The vector generated, in which the newly introduced expression cassette is orientated in the opposite direction as the GAPD-GNE cassette, is designated pSH872. Next, the 2.2 Kb expression cassette containing the TEF-ST is digested with *Sgf*I from pSH893 and subcloned into pSH872 digested with the same enzyme. The vector generated, in which the TEF-STcassette-orientated in the opposite direction as the GAPDH-GNE cassette,is designated pSH926.

The pSH926 vector is transformed into strain YSH661, which is then capable of producing rat EPO having NANA₂Gal₂GlcNAc₂Man₃GlcNAc₂ *N*-glycans.

### EXAMPLE 3

A *Pichia pastoris* strain capable of producing a human EPO having NANA₂Gal₂GlcNAc₂Man₃GlcNAc₂(Fuc) *N*-glycans can be made by introducing the vector pSH1022 into a *Pichia pastoris* strain capable of producing human EPO having NANA₂Gal₂GlcNAc₂Man₃GlcNAc₂ *N*-glycans. For example, vector pSH1022 containing the genes encoding the components of the fucosylation pathway can be transformed into a strain that is capable of producing glycoproteins having NANA₂Gal₂GlcNAc₂Man₃GlcNAc₂:N-glycans; such as strain YSN59.7 disclosed in Hamilton et al., Science 313, 1441-1443 (2006) or YSH661 of Example 2 comprising the genes encoding the sialylation pathway enzymes but replacing the DNA encoding rat EPO with DNA encoding the human EPO. The strain will then produce human EPO having NANA₂Gal₂GleNAc₂Man₃GlcNAc₂(Fuc) N-glycans.

### SEQUENCES

SEQ ID NO: 1
   DNA
   Homo sapian
   GDP-Mannose-dehydratase (hGMD)
SEQ ID NO:2
   Protein
   Homo sapian
   GDP-Mannose-dehydratase (hGMD)
SEQ ID NO:3
   DNA
   Homo sapian
   GDP-keto-deoxy-mannose-epimerase/GDP-keto-deoxy-galactose-reductase (FX protein)
SEQ ID NO:4 .
   Protein
   Homo sapian
   GDP-keto-deoxy-mannose-epimerase/GDP-keto-deoxy-galactose-reductase (FX protein)
SEQIDNO:5
   DNA
   Homo sapian
   Human GDP-fucose transporter
SEQ ID NO:6
   Protein
   Homo sapian
   Human GDP-fucose transporter
SEQ ID NO:7
   DNA
   Homo sapian
   alpha 1,6 fucosyltransferase (hFuT8)
SEQ ID NO:8
   Protein
   Homo sapian
   alpha 1,6 fucosyltransferase (hFuT8)
SEQ ID NO:9
   DNA
   Rat
   Alpha-1,6-fucosyltransferase (rFuT8)
SEQ ID NO: *10*
   Protein
   Rat
   Alpha-1,6-fucosyltransferase (rFuT8)
SEQ ID NO:11
   DNA
   Mouse
   Alpha-1,6-fucosyltransferase (mFuT8)
SEQ ID NO:12
   Protein
   Mouse
   Alpha-1,6-fucosyltransferase (mFuT8)
SEQ ID NO:13
   DNA
   Porcine
   Alpha-1,6-fucosyltransferase (pFuT8)
SEQ ID NO:13
   Protein
   Porcine
   Alpha-1,6-fucosyltransferase (pFuT8)
SEQ ID NO:15
   DNA
   Artificial
   PCR primer SH415
   GGCGG CCGCC ACCAT GGCAC ACGCA CCGGC ACGCT GC
SEQ ID NO:16
   DNA
   Artificial
   PCR primer SH413
   TTAAT TAATC AGGCA TTGGG GTTTG TCCTC ATG
SEQ ID NO:17
   DNA
   Artificial
   PCR primer SH414
   GGCGG CCGCC ACCAT GGGTG AACCC CAGGG ATCCA TG
SEQ ID NO:18
   DNA
   Artificial
   PCR primer SH411
   TTAAT TAATC ACTTC CGGGC CTGCT CGTAG TTG
SEQ ID NO:19
   DNA
   Artificial
   PCR primer RCD679
   GCGGC CGCCA CCATG AATAG GGCCC CTCTG AAGCG G
SEQ ID NO:20
   DNA
   Artificial
   PCR primer RCD680
   TTAAT TAATC ACACC CCCAT GGCGC TCTTC TC
SEQ B7 NO:21
   DNA
   Artificial
   PCR primer SH420
   GCGGC GCGCC GATAA TGACC ACCCT GATCA CTCCA G
SEQ ID NO:22
   DNA
   Artificial
   PCR primer SH421
   CCTTA ATTAA CTATT TTTCA GCTTC AGGAT ATGTG GG-
SEQ ID NO:23
   DNA
   Artificial
   PCR primer rEPO-forward
   GGGAA TTCGC TCCCC CACGC CTCAT TTGCG AC
SEQ ID NO:24
   DNA
   Artificial
   PCR primer rEPO-reverse
   CCTCT AGATC ACCTG TCCCC TCTCC TGCAG GC
SEQ ID NO:25
   DNA
   Artificial
   PCR primer ScMnt1-for
   GGGCGGCCGCCACCATGGCCCTCTTTCTC AGTAAGAGACT GTTGAG
SEQ ID NO:26
   DNA
   Artificial
   PCR primer ScMnt1-rev
   CCGGCGCGCCCGATGACTTGTTG TTCAGGGGATATAGATCCTG

## Claims

1. A recombinant *Pichia pastoris* host cell comprising a fucosylation pathway wherein the enzymes and activities comprising the pathway are provided by nucleic acids encoding a GDP-mannose-dehydratase (GMD), a GDP-keto-deoxy-mannose-epimerase/GDP-keto-deoxy-galactose-reductase (FX), a GDP-Fucose transporter (GFTr), and a fusion protein that comprises the catalytic domain of α1,6-fucosyltransferase EC 2.4.1.68 fused to amino acids 1-36 of the *S*. *cerevisiae Mnn2*.

2. The host cell of claim 1 wherein the host cell further does not display α1,6-mannosyltransferase activity with respect to the N-glycan on a glycoprotein and includes an α1,2-mannosidase catalytic domain fused to a cellular targeting signal peptide not normally associated with the catalytic domain and selected to target α1,2-mannosidase activity to the ER or Golgi apparatus of the host cell whereby, upon passage of a recombinant glycoprotein through the ER or Golgi apparatus of the host cell, a recombinant glycoprotein comprising a fucosylated Man₅GlcNAc₂ glycoform is produced.

3. The host cell of Claim 2 further including a GlcNAc transferase I catalytic domain fused to a cellular targeting signal peptide not normally associated with the catalytic domain of and selected to target GlcNAc transferase I activity to the ER or Golgi apparatus of the host cell; whereby, upon passage of the recombinant glycoprotein through the ER or Golgi apparatus of the host cell, a recombinant glycoprotein comprising a fucosylated GlcNAcMan₅GlcNAc₂ glycoform is produced.

4. The host cell of Claim 3 further including a mannosidase II catalytic domain fused to a cellular targeting signal peptide not normally associated with the catalytic domain and selected to target mannosidase II activity to the ER or Golgi apparatus of the host cell; whereby, upon passage of the recombinant glycoprotein through the ER or Golgi apparatus of the host cell, a recombinant glycoprotein comprising a fucosylated GlcNAcMan₃GlcNAc₂ glycoform is produced.

5. The host cell of Claim 4 further including a GlcNAc transferase II catalytic domain fused to a cellular targeting signal peptide not normally associated with the catalytic domain and selected to target GlcNAc transferase II activity to the ER or Golgi apparatus of the host cell; whereby, upon passage of the recombinant glycoprotein through the ER or Golgi apparatus of the host cell, a recombinant glycoprotein comprising a fucosylated GlcNAc₂Man₃GlcNAc₂ glycoform is produced.

6. The host cell of Claim 5 further including a Galactose transferase II catalytic domain fused to a cellular targeting signal peptide not normally associated with the catalytic domain and selected to target Galactose transferase II activity to the ER or Golgi apparatus of the host cell; whereby, upon passage of the recombinant glycoprotein through the ER or Golgi apparatus of the host cell, a recombinant glycoprotein comprising a fucosylated Gal₂GlcNAc₂Man₃GlcNAc₂ glycoform is produced.

7. The host cell of Claim 6 further including a sialyltransferase catalytic domain fused to a cellular targeting signal peptide not normally associated with the catalytic domain and selected to target sialyltransferase activity to the ER or Golgi apparatus of the host cell; whereby, upon passage of the recombinant glycoprotein through the ER or Golgi apparatus of the host cell, a recombinant glycoprotein comprising a fucosylated NANA₂Gal₂GlcNAc₂Man₃GlcNAc₂ glycoform is produced.

8. A hybrid vector comprising (a) DNA regulatory elements which are functional in a lower eukaryotic host cell operatively linked to (b) a DNA coding sequence encoding a fusion protein comprising amino acids 1-36 of the S. *cerevisiae Mnn2* fused to the catalytic domain of α1,6-fucosyltransferase EC 2.4.1.68.

## Patentansprüche

1. Eine rekombinante Pichia-pastoris-Wirtszelle, die einen Fucosylierungsweg umfasst, wobei die Enzyme und Aktivitäten, die den Weg bilden, von Nukleinsäuren bereitgestellt werden, die eine GDP-Mannose-Dehydratase (GMD), eine GDP-Ketodesoxymannose-Epimerase/GDP-Ketodesoxygalactose-Reduktase (FX), einen GDP-Fucose-Transporter (GFTr) und ein Fusionsprotein, das die katalytische Domäne von α1,6-Fucosyltransferase EC 2.4.1.68, fusioniert mit den Aminosäuren 1-36 von S. *cerevisiae Mnn2,* umfasst, kodieren.

2. Die Wirtszelle nach Anspruch 1, wobei die Wirtszelle ferner keine α1,6-Mannosyltransferase-Aktivität hinsichtlich des *N*-Glycans an einem Glycoprotein aufweist und eine katalytische Domäne der α1,2-Mannosidase fusioniert mit einem Zell-Targeting-Signalpeptid umfasst, das normalerweise nicht mit der katalytischen Domäne assoziiert ist und ausgewählt ist, um die α1,2-Mannosidase-Aktivität zum ER oder Golgi-Apparat der Wirtszelle zu steuern, wobei beim Durchgang eines rekombinanten Glycoproteins durch das ER oder den Golgi-Apparat der Wirtszelle ein rekombinantes Glycoprotein erzeugt wird, das eine fucosylierte Man₅GlcNAc₂-Glycoform umfasst.

3. Die Wirtszelle nach Anspruch 2, ferner umfassend eine katalytische Domäne der GlcNAc-Transferase I, die mit einem Zell-Targeting-Signalpeptid fusioniert ist, das normalerweise nicht mit der katalytischen Domäne assoziiert ist und ausgewählt ist, um die GlcNAc-Transferase-I-Aktivität zum ER oder Golgi-Apparat der Wirtszelle zu steuern, wobei beim Durchgang des rekombinanten Glycoproteins durch das ER oder den Golgi-Apparat der Wirtszelle ein rekombinantes Glycoprotein erzeugt wird, das eine fucosylierte GlcNAcMan₅GlcNAc₂-Glycoform umfasst.

4. Die Wirtszelle nach Anspruch 3, ferner umfassend eine katalytische Domäne der Mannosidase II, die mit einem Zell-Targeting-Signalpeptid fusioniert ist, das normalerweise nicht mit der katalytischen Domäne assoziiert ist und ausgewählt ist, um die Mannosidase-II-Aktivität zum ER oder Golgi-Apparat der Wirtszelle zu steuern, wobei beim Durchgang des rekombinanten Glycoproteins durch das ER oder den Golgi-Apparat der Wirtszelle ein rekombinantes Glycoprotein erzeugt wird, das eine fucosylierte GlcNAcMan₃GlcNAc₂-Glycoform umfasst.

5. Die Wirtszelle nach Anspruch 4, ferner umfassend eine katalytische Domäne der GlcNAc-Transferase II, die mit einem Zell-Targeting-Signalpeptid fusioniert ist, das normalerweise nicht mit der katalytischen Domäne assoziiert ist und ausgewählt ist, um die GlcNAc-Transferase-II-Aktivität zum ER oder Golgi-Apparat der Wirtszelle zu steuern, wobei beim Durchgang des rekombinanten Glycoproteins durch das ER oder den Golgi-Apparat der Wirtszelle ein rekombinantes Glycoprotein erzeugt wird, das eine fucosylierte GlcNAC₂Man₃GlcNAC₂-Glycoform umfasst.

6. Die Wirtszelle nach Anspruch 5, ferner umfassend eine katalytische Domäne der Galactose-Transferase II, die mit einem Zell-Targeting-Signalpeptid fusioniert ist, das normalerweise nicht mit der katalytischen Domäne assoziiert ist und ausgewählt ist, um die Galactose-Transferase-II-Aktivität zum ER oder Golgi-Apparat der Wirtszelle zu steuern, wobei beim Durchgang des rekombinanten Glycoproteins durch das ER oder den Golgi-Apparat der Wirtszelle ein rekombinantes Glycoprotein erzeugt wird, das eine fucosylierte Gal₂GlcNAc₂Man₃GlcNAc₂-Glycoform umfasst.

7. Die Wirtszelle nach Anspruch 6, ferner umfassend eine katalytische Domäne der Sialyltransferase, die mit einem Zell-Targeting-Signalpeptid fusioniert ist, das normalerweise nicht mit der katalytischen Domäne assoziiert ist und ausgewählt ist, um die Sialyltransferase-Aktivität zum ER oder Golgi-Apparat der Wirtszelle zu steuern, wobei beim Durchgang des rekombinanten Glycoproteins durch das ER oder den Golgi-Apparat der Wirtszelle ein rekombinantes Glycoprotein erzeugt wird, das eine fucosylierte NANA₂Gal₂GlcNAc₂Man₃GlcNAc₂-Glycoform umfasst.

8. Ein Hybridvektor, umfassend (a) DNA-regulatorische Elemente, die in einer niederen eukaryotischen Wirtszelle funktionieren, operativ verbunden mit (b) einer kodierenden DNA-Sequenz, die ein Fusionsprotein kodiert, umfassend die Aminosäuren 1-36 von S. *cerevisiae Mnn2,* fusioniert mit der katalytischen Domäne von α1,6-Fucosyltransferase EC 2.4.1.68.

## Revendications

1. Cellule hôte recombinante de *Pichia pastoris* comprenant une voie de fucosylation dans laquelle les enzymes et les activités comprenant la voie sont fournies par des acides nucléiques codant une GDP-mannose-déshydratase (GMD), une GDP-céto-désoxy-mannose-épimérase/GDP-céto-désoxy-galactose-réductase (FX, un GDP-transporteur de fucose (GFTr) et une protéine de fusion qui comprend le domaine catalytique de la α1,6-fucosyltransférase EC 2.4.1.68 fusionné aux acides aminés 1-36 du *Mnn2* de *S*. *cerevisiae.*

2. Cellule hôte selon la revendication 1, où en outre la cellule hôte ne présente pas d'activité α1,6-mannosyltransférase en ce qui concerne le *N*-glycane sur une glycoprotéine qui comprend un domaine catalytique de α1,2-mannosidase fusionné à un peptide signal de ciblage cellulaire non associé normalement au domaine catalytique et sélectionné pour cibler une activité α1,2-mannosidase sur le RE ou l'appareil de Golgi de la cellule hôte; en vertu de quoi, lors du passage d'une glycoprotéine recombinante à travers le RE ou l'appareil de Golgi de la cellule hôte, une glycoprotéine recombinante comprenant une glycoforme Man₅GlcNac₂ fucosylée est produite.

3. Cellule hôte selon la revendication 2, comprenant en outre un domaine catalytique de GlcNAc transférase 1 fusionné à un peptide signal de ciblage cellulaire non associé normalement au domaine catalytique et sélectionné pour cibler l'activité GlcNAc transférase I sur le RE ou l'appareil de Golgi de la cellule hôte; en vertu de quoi, lors du passage de la glycoprotéine recombinante à travers le RE ou l'appareil de Golgi de la cellule hôte, une glycoprotéine recombinante comprenant une glycoforme GlcNAcMan₅GlcNAc₂ fucosylée est produite.

4. Cellule hôte selon la revendication 3, comprenant en outre un domaine catalytique de mannosidase II fusionné à un peptide signal de ciblage cellulaire non associé normalement au domaine catalytique et sélectionné pour cibler l'activité mannosidase II sur le RE ou l'appareil de Golgi de la cellule hôte; en vertu de quoi, lors du passage de la glycoprotéine recombinante à travers le RE ou l'appareil de Golgi de la cellule hôte, une glycoprotéine recombinante comprenant une glycoforme GlcNAcMan₃GlcNAc₂ fucosylée est produite.

5. Cellule hôte selon la revendication 4, comprenant en outre un domaine catalytique de GlcNAc transférase II fusionné à un peptide signal de ciblage cellulaire non associé normalement au domaine catalytique et sélectionné pour cibler l'activité GlcNAc transférase II sur le RE ou l'appareil de Golgi de la cellule hôte; en vertu de quoi, lors du passage de la glycoprotéine recombinante à travers le RE ou l'appareil de Golgi de la cellule hôte, une glycoprotéine recombinante comprenant une glycoforme GlcNAc₂Man₃GlcNAc₂ fucosylée est produite.

6. Cellule hôte selon la revendication 5, comprenant en outre un domaine catalytique de Galactose transférase II fusionné à un peptide signal de ciblage cellulaire non associé normalement au domaine catalytique et sélectionné pour cibler l'activité Galactose transférase II sur le RE ou l'appareil de Golgi de la cellule hôte; en vertu de quoi, lors du passage de la glycoprotéine recombinante à travers le RE ou l'appareil de Golgi de la cellule hôte, une glycoprotéine recombinante comprenant une glycoforme Gal₂GlcNAc₂Man₃GlcNAc₂ fucosylée est produite.

7. Cellule hôte selon la revendication 6, comprenant en outre un domaine catalytique de sialyltransférase fusionné à un peptide signal de ciblage cellulaire non associé normalement au domaine catalytique et sélectionné pour cibler l'activité sialyltransférase sur le RE ou l'appareil de Golgi de la cellule hôte; en vertu de quoi, lors du passage de la glycoprotéine recombinante à travers le RE ou l'appareil de Golgi de la cellule hôte, une glycoprotéine recombinante comprenant une glycoforme NANA₂Gal₂GlcNAc₂Man₃GlcNAc₂ fucosylée est produite.

8. Vecteur hybride comprenant (a) des éléments régulateurs d'ADN qui sont fonctionnels dans une cellule hôte eucaryote inférieure, liés d'une manière opérationnelle à (b) une séquence codante d'ADN codant une protéine de fusion comprenant les acides aminés 1-36 du *Mnn2* de *S. cerevisiae* fusionnés au domaine catalytique de la α1,6-fucosyltransférase EC 2.4.1.68.


### SEQUENCE LISTING

<110> Stephen Hamilton
<120> PRODUCTION OF GLYCOPROTEINS WITH MODIFIED FUCOSYLATION
<130> GF0028Y
<150> 60/905,345
   <151> 2007-03-07
<160> 26
<170> FastSEQ for Windows Version 4.0
<210> 1
   <211> 1119
   <212> DNA
   <213> Homo sapians
<220>
   <221> CDS
   <222> (1)...(1119)
   <223> GDP-Mannose-dehydratase (hGMD)
<400> 1
   atg gca cac gca ccg gca cgc tgc ccc agc gcc cgg ggc tcc ggg gac    48
   Met Ala His Ala Pro Ala Arg Cys Pro Ser Ala Arg Gly Ser Gly Asp
    1               5                  10                  15
   ggc gag atg ggc aag ccc agg aac gtg gcg ctc atc acc ggt atc aca    96
   Gly Glu Met Gly Lys Pro Arg Asn Val Ala Leu Ile Thr Gly Ile Thr
               20                  25                  30
   ggc cag gat ggt tcc tac ctg gct gag ttc ctg ctg gag aaa ggc tat   144
   Gly Gln Asp Gly Ser Tyr Leu Ala Glu Phe Leu Leu Glu Lys Gly Tyr
           35                  40                  45
   gag gtc cat gga att gta cgg cgg tcc agt tca ttt aat acg ggt cga   192
   Glu Val His Gly Ile Val Arg Arg Ser Ser Ser Phe Asn Thr Gly Arg
       50                  55                  60
   att gag cat ctg tat aag aat ccc cag gct cac att gaa gga aac atg   240
   Ile Glu His Leu Tyr Lys Asn Pro Gln Ala His Ile Glu Gly Asn Met
   65                  70                  75                  80
   aag ttg cac tat ggc gat ctc act gac agt acc tgc ctt gtg aag atc   288
   Lys Leu His Tyr Gly Asp Leu Thr Asp Ser Thr Cys Leu Val Lys Ile
                   85                  90                  95
   att aat gaa gta aag ccc aca gag atc tac aac ctt gga gcc cag agc   336
   Ile Asn Glu Val Lys Pro Thr Glu Ile Tyr Asn Leu Gly Ala Gln Ser
               100                 105                 110
   cac gtc aaa att tcc ttt gac ctc gct gag tac act gcg gac gtt gac   384
   His Val Lys Ile Ser Phe Asp Leu Ala Glu Tyr Thr Ala Asp Val Asp
           115                 120                 125
   gga gtt ggc act cta cga ctt cta gat gca gtt aag act tgt ggc ctt   432
   Gly Val Gly Thr Leu Arg Leu Leu Asp Ala Val Lys Thr Cys Gly Leu
       130                 135                 140
   atc aac tct gtg aag ttc tac caa gcc tca aca agt gaa ctt tat ggg   480
   Ile Asn Ser Val Lys Phe Tyr Gln Ala Ser Thr Ser Glu Leu Tyr Gly
   145                 150                 155                 160
   aaa gtg cag gaa ata ccc cag aag gag acc acc cct ttc tat ccc cgg      528
   Lys Val Gln Glu Ile Pro Gln Lys Glu Thr Thr Pro Phe Tyr Pro Arg
                   165                 170                 175
   tca ccc tat ggg gca gca aaa ctc tat gcc tat tgg att gtg gtg aac      576
   Ser Pro Tyr Gly Ala Ala Lys Leu Tyr Ala Tyr Trp Ile Val Val Asn
               180                 185                 190
   ttc cgt gag gcg tat aat ctc ttt gca gtg aac ggc att ctc ttc aat      624
   Phe Arg Glu Ala Tyr Asn Leu Phe Ala Val Asn Gly Ile Leu Phe Asn
           195                 200                 205
   cat gag agt ccc aga aga gga gct aat ttc gtt act cga aaa att agc      672
   His Glu Ser Pro Arg Arg Gly Ala Asn Phe Val Thr Arg Lys Ile Ser
       210                 215                 220
   cgg tca gta gct aag att tac ctt gga caa ctg gaa tgt ttc agt ttg      720
   Arg Ser Val Ala Lys Ile Tyr Leu Gly Gln Leu Glu Cys Phe Ser Leu
   225                 230                 235                 240
   gga aat ctg gat gcc aaa cga gat tgg ggc cat gcc aag gac tat gtg      768
   Gly Asn Leu Asp Ala Lys Arg Asp Trp Gly His Ala Lys Asp Tyr Val
                   245                 250                 255
   gag gct atg tgg ttg atg ttg cag aat gat gag ccg gag gac ttc gtt      816
   Glu Ala Met Trp Leu Met Leu Gln Asn Asp Glu Pro Glu Asp Phe Val
               260                 265                 270
   ata gct act ggg gag gtc cat agt gtc cgg gaa ttt gtc gag aaa tca      864
   Ile Ala Thr Gly Glu Val His Ser Val Arg Glu Phe Val Glu Lys Ser
           275                 280                 285
   ttc ttg cac att gga aaa acc att gtg tgg gaa gga aag aat gaa aat      912
   Phe Leu His Ile Gly Lys Thr Ile Val Trp Glu Gly Lys Asn Glu Asn
       290                 295                 300
   gaa gtg ggc aga tgt aaa gag acc ggc aaa gtt cac gtg act gtg gat      960
   Glu Val Gly Arg Cys Lys Glu Thr Gly Lys Val His Val Thr Val Asp
   305                 310                 315                 320
   ctc aag tac tac cgg cca act gaa gtg gac ttt ctg cag ggc gac tgc     1008
   Leu Lys Tyr Tyr Arg Pro Thr Glu Val Asp Phe Leu Gln Gly Asp Cys
                   325                 330                 335
   acc aaa gcg aaa cag aag ctg aac tgg aag ccc cgg gtc gct ttc gat     1056
   Thr Lys Ala Lys Gln Lys Leu Asn Trp Lys Pro Arg Val Ala Phe Asp
               340                 345                 350
   gag ctg gtg agg gag atg gtg cac gcc gac gtg gag ctc atg agg aca     1104
   Glu Leu Val Arg Glu Met Val His Ala Asp Val Glu Leu Met Arg Thr
           355                 360                 365
   aac ccc aat gcc tga                                                 1119
   Asn Pro Asn Ala  *
       370
<210> 2
   <211> 372
   <212> PRT
   <213> Homo sapians
<400> 2
   Met Ala His Ala Pro Ala Arg Cys Pro Ser Ala Arg Gly Ser Gly Asp
    1               5                  10                  15
   Gly Glu Met Gly Lys Pro Arg Asn Val Ala Leu Ile Thr Gly Ile Thr
               20                  25                  30
   Gly Gln Asp Gly Ser Tyr Leu Ala Glu Phe Leu Leu Glu Lys Gly Tyr
           35                  40                  45
   Glu Val His Gly Ile Val Arg Arg Ser Ser Ser Phe Asn Thr Gly Arg
       50                  55                  60
   Ile Glu His Leu Tyr Lys Asn Pro Gln Ala His Ile Glu Gly Asn Met
   65                  70                  75                  80
   Lys Leu His Tyr Gly Asp Leu Thr Asp Ser Thr Cys Leu Val Lys Ile
                   85                  90                  95
   Ile Asn Glu Val Lys Pro Thr Glu Ile Tyr Asn Leu Gly Ala Gln Ser
               100                 105                 110
   His Val Lys Ile Ser Phe Asp Leu Ala Glu Tyr Thr Ala Asp Val Asp
           115                 120                 125
   Gly Val Gly Thr Leu Arg Leu Leu Asp Ala Val Lys Thr Cys Gly Leu
       130                 135                 140
   Ile Asn Ser Val Lys Phe Tyr Gln Ala Ser Thr Ser Glu Leu Tyr Gly
   145                 150                 155                 160
   Lys Val Gln Glu Ile Pro Gln Lys Glu Thr Thr Pro Phe Tyr Pro Arg
                   165                 170                 175
   Ser Pro Tyr Gly Ala Ala Lys Leu Tyr Ala Tyr Trp Ile Val Val Asn
               180                 185                 190
   Phe Arg Glu Ala Tyr Asn Leu Phe Ala Val Asn Gly Ile Leu Phe Asn
           195                 200                 205
   His Glu Ser Pro Arg Arg Gly Ala Asn Phe Val Thr Arg Lys Ile Ser
       210                 215                 220
   Arg Ser Val Ala Lys Ile Tyr Leu Gly Gln Leu Glu Cys Phe Ser Leu
   225                 230                 235                 240
   Gly Asn Leu Asp Ala Lys Arg Asp Trp Gly His Ala Lys Asp Tyr Val
                   245                 250                 255
   Glu Ala Met Trp Leu Met Leu Gln Asn Asp Glu Pro Glu Asp Phe Val
               260                 265                 270
   Ile Ala Thr Gly Glu Val His Ser Val Arg Glu Phe Val Glu Lys Ser
           275                 280                 285
   Phe Leu His Ile Gly Lys Thr Ile Val Trp Glu Gly Lys Asn Glu Asn
       290                 295                 300
   Glu Val Gly Arg Cys Lys Glu Thr Gly Lys Val His Val Thr Val Asp
   305                 310                 315                 320
   Leu Lys Tyr Tyr Arg Pro Thr Glu Val Asp Phe Leu Gln Gly Asp Cys
                   325                 330                 335
   Thr Lys Ala Lys Gln Lys Leu Asn Trp Lys Pro Arg Val Ala Phe Asp
               340                 345                 350
   Glu Leu Val Arg Glu Met Val His Ala Asp Val Glu Leu Met Arg Thr
           355                 360                 365
   Asn Pro Asn Ala
       370
<210> 3
   <211> 966
   <212> DNA
   <213> Homo sapians
<220>
   <221> CDS
   <222> (1)...(966)
   <223> GDP-ketoxy-deoxy-mannose-epimerase/GDP-keto-deoxy-galactose-reductase (FX protein)
<400> 3
   atg ggt gaa ccc cag gga tcc atg cgg att cta gtg aca ggg ggc tct   48
   Met Gly Glu Pro Gln Gly Ser Met Arg Ile Leu Val Thr Gly Gly Ser
   1 5 10 15
   ggg ctg gta ggc aaa gcc atc cag aag gtg gta gca gat gga gct gga 96
   Gly Leu Val Gly Lys Ala Ile Gln Lys Val Val Ala Asp Gly Ala Gly
   20 25 30
   ctt cct gga gag gac tgg gtg ttt gtc tcc tct aaa gac gcc gat ctc 144
   Leu Pro Gly Glu Asp Trp Val Phe Val Ser Ser Lys Asp Ala Asp Leu
   35 40 45
   acg gat aca gca cag acc cgc gcc ctg ttt gag aag gtc caa ccc aca 192
   Thr Asp Thr Ala Gln Thr Arg Ala Leu Phe Glu Lys Val Gln Pro Thr
   50 55 60
   cac gtc atc cat ctt gct gca atg gtg ggg ggc ctg ttc cgg aat atc 240
   His Val Ile His Leu Ala Ala Met Val Gly Gly Leu Phe Arg Asn Ile
   65 70 75 80
   aaa tac aat ttg gac ttc tgg agg aaa aac gtg cac atg aac gac aac 288
   Lys Tyr Asn Leu Asp Phe Trp Arg Lys Asn Val His Met Asn Asp Asn
   85 90 95
   gtc ctg cac tcg gcc ttt gag gtg ggg gcc cgc aag gtg gtg tcc tgc 336
   Val Leu His Ser Ala Phe Glu Val Gly Ala Arg Lys Val Val Ser Cys
   100 105 110
   ctg tcc acc tgt atc ttc cct gac aag acg acc tac ccg ata gat gag 384
   Leu Ser Thr Cys Ile Phe Pro Asp Lys Thr Thr Tyr Pro Ile Asp Glu
   115 120 125
   acc atg atc cac aat ggg cct ccc cac aac agc aat ttt ggg tac tcg 432
   Thr Met Ile His Asn Gly Pro Pro His Asn Ser Asn Phe Gly Tyr Ser
   130 135 140
   tat gcc aag agg atg atc gac gtg cag aac agg gcc tac ttc cag cag 480
   Tyr Ala Lys Arg Met Ile Asp Val Gln Asn Arg Ala Tyr Phe Gln Gln
   145 150 155 160
   tac ggc tgc acc ttc acc gct gtc atc ccc acc aac gtt ttc ggg ccc 528
   Tyr Gly Cys Thr Phe Thr Ala Val Ile Pro Thr Asn Val Phe Gly Pro
   165 170 175
   cac gac aac ttc aac atc gag gat ggc cac gtg ctg cct ggc ctc atc 576
   His Asp Asn Phe Asn Ile Glu Asp Gly His Val Leu Pro Gly Leu Ile
   180 185 190
   cac aag gtg cac ctg gcc aag agc agc ggc tcg gcc ctg acg gtg tgg 624
   His Lys Val His Leu Ala Lys Ser Ser Gly Ser Ala Leu Thr Val Trp
   195 200 205
   ggt aca ggg aat ccg cgg agg cag ttc ata tac tcg ctg gac ctg gcc 672
   Gly Thr Gly Asn Pro Arg Arg Gln Phe Ile Tyr Ser Leu Asp Leu Ala
   210 215 220
   cag ctc ttt atc tgg gtc ctg cgg gag tac aat gaa gtg gag ccc atc 720
   Gln Leu Phe Ile Trp Val Leu Arg Glu Tyr Asn Glu Val Glu Pro Ile
   225 230 235 240
   atc ctc tcc gtg ggc gag gaa gat gag gtc tcc atc aag gag gca gcc 768
   Ile Leu Ser Val Gly Glu Glu Asp Glu Val Ser Ile Lys Glu Ala Ala
   245 250 255
   gag gcg gtg gtg gag gcc atg gac ttc cat ggg gaa gtc acc ttt gat 816
   Glu Ala Val Val Glu Ala Met Asp Phe His Gly Glu Val Thr Phe Asp
   260 265 270
   aca acc aag tcg gat ggg cag ttt aag aag aca gcc agt aac agc aag    864
   Thr Thr Lys Ser Asp Gly Gln Phe Lys Lys Thr Ala Ser Asn Ser Lys
           275                 280                 285
   ctg agg acc tac ctg ccc gac ttc cgg ttc aca ccc ttc aag cag gcg    912
   Leu Arg Thr Tyr Leu Pro Asp Phe Arg Phe Thr Pro Phe Lys Gln Ala
       290                 295                 300
   gtg aag gag acc tgt gct tgg ttc act gac aac tac gag cag gcc cgg    960
   Val Lys Glu Thr Cys Ala Trp Phe Thr Asp Asn Tyr Glu Gln Ala Arg
   305                 310                 315                 320
   aag tga                                                            966
   Lys  *
<210> 4
   <211> 321
   <212> PRT
   <213> Homo sapians
<400> 4
   Met Gly Glu Pro Gln Gly Ser Met Arg Ile Leu Val Thr Gly Gly Ser
    1               5                  10                  15
   Gly Leu Val Gly Lys Ala Ile Gln Lys Val Val Ala Asp Gly Ala Gly
               20                  25                  30
   Leu Pro Gly Glu Asp Trp Val Phe Val Ser Ser Lys Asp Ala Asp Leu
           35                  40                  45
   Thr Asp Thr Ala Gln Thr Arg Ala Leu Phe Glu Lys Val Gln Pro Thr
       50                  55                  60
   His Val Ile His Leu Ala Ala Met Val Gly Gly Leu Phe Arg Asn Ile
   65                  70                  75                  80
   Lys Tyr Asn Leu Asp Phe Trp Arg Lys Asn Val His Met Asn Asp Asn
                   85                  90                  95
   Val Leu His Ser Ala Phe Glu Val Gly Ala Arg Lys Val Val Ser Cys
               100                 105                 110
   Leu Ser Thr Cys Ile Phe Pro Asp Lys Thr Thr Tyr Pro Ile Asp Glu
           115                 120                 125
   Thr Met Ile His Asn Gly Pro Pro His Asn Ser Asn Phe Gly Tyr Ser
       130                 135                 140
   Tyr Ala Lys Arg Met Ile Asp Val Gln Asn Arg Ala Tyr Phe Gln Gln
   145                 150                 155                 160
   Tyr Gly Cys Thr Phe Thr Ala Val Ile Pro Thr Asn Val Phe Gly Pro
                   165                 170                 175
   His Asp Asn Phe Asn Ile Glu Asp Gly His Val Leu Pro Gly Leu Ile
               180                 185                 190
   His Lys Val His Leu Ala Lys Ser Ser Gly Ser Ala Leu Thr Val Trp
           195                 200                 205
   Gly Thr Gly Asn Pro Arg Arg Gln Phe Ile Tyr Ser Leu Asp Leu Ala
       210                 215                 220
   Gln Leu Phe Ile Trp Val Leu Arg Glu Tyr Asn Glu Val Glu Pro Ile
   225                 230                 235                 240
   Ile Leu Ser Val Gly Glu Glu Asp Glu Val Ser Ile Lys Glu Ala Ala
                   245                 250                 255
   Glu Ala Val Val Glu Ala Met Asp Phe His Gly Glu Val Thr Phe Asp
               260                 265                 270
   Thr Thr Lys Ser Asp Gly Gln Phe Lys Lys Thr Ala Ser Asn Ser Lys
           275                 280                 285
   Leu Arg Thr Tyr Leu Pro Asp Phe Arg Phe Thr Pro Phe Lys Gln Ala
       290                 295                 300
   Val Lys Glu Thr Cys Ala Trp Phe Thr Asp Asn Tyr Glu Gln Ala Arg
   305                 310                 315                 320
   Lys
<210> 5
   <211> 1095
   <212> DNA
   <213> Homo sapians
<220>
   <221> CDS
   <222> (1)...(1095)
   <223> GDP-fucose transporter
<400> 5
atg aat agg gcc cct ctg aag cgg tcc agg atc ctg cac atg gcg ctg    48
   acc ggg gcc tca gac ccc tct gca gag gca gag gcc aac ggg gag aag    96
   ccc ttt ctg ctg cgg gca ttg cag atc gcg ctg gtg gtc tcc ctc tac    144
   tgg gtc acc tcc atc tcc atg gtg ttc ctt aat aag tac ctg ctg gac    192
   agc ccc tcc ctg cgg ctg gac acc ccc atc ttc gtc acc ttc tac cag    240
   tgc ctg gtg acc acg ctg ctg tgc aaa ggc ctc agc gct ctg gcc gcc    288
   tgc tgc cct ggt gcc gtg gac ttc ccc agc ttg cgc ctg gac ctc agg    336
   gtg gcc cgc agc gtc ctg ccc ctg tcg gtg gtc ttc atc ggc atg atc    384
   acc ttc aat aac ctc tgc ctc aag tac gtc ggt gtg gcc ttc tac aat    432
   gtg ggc cgc tca ctc acc acc gtc ttc aac gtg ctg ctc tcc tac ctg    480
   ctg ctc aag cag acc acc tcc ttc tat gcc ctg ctc acc tgc ggt atc    528
   atc atc ggg ggc ttc tgg ctt ggt gtg gac cag gag ggg gca gaa ggc    576
   acc ctg tcg tgg ctg ggc acc gtc ttc ggc gtg ctg gct agc ctc tgt    624
   gtc tcg ctc aac gcc atc tac acc acg aag gtg ctc ccg gcg gtg gac    672
   ggc agc atc tgg cgc ctg act ttc tac aac aac gtc aac gcc tgc atc    720
   ctc ttc ctg ccc ctg ctc ctg ctg ctc ggg gag ctt cag gcc ctg cgt    768
   gac ttt gcc cag ctg ggc agt gcc cac ttc tgg ggg atg atg acg ctg    816
   ggc ggc ctg ttt ggc ttt gcc atc ggc tac gtg aca gga ctg cag atc    864
   aag ttc acc agt ccg ctg acc cac aat gtg tcg ggc acg gcc aag gcc    912
   tgt gcc cag aca gtg ctg gcc gtg ctc tac tac gag gag acc aag agc    960
   ttc ctc tgg tgg acg agc aac atg atg gtg ctg ggc ggc tcc tcc gcc    1008
   tac acc tgg gtc agg ggc tgg gag atg aag aag act ccg gag gag ccc    1056
   agc ccc aaa gac agc gag aag agc gcc atg ggg gtg tga                1095
<210> 6
   <211> 364
   <212> PRT
   <213> Homo sapians
<400> 6
<210> 7
   <211> 1728
   <212> DNA
   <213> Homo sapians
<220>
   <221> CDS
   <222> (1)...(1728)
   <223> alpha-1,6-fucosyltransferase (hFuT8)
<400> 7
   atg cgg cca tgg act ggt tcc tgg cgt tgg att atg ctc att ctt ttt    48
   Met Arg Pro Trp Thr Gly Ser Trp Arg Trp Ile Met Leu Ile Leu Phe
    1               5                  10                  15
   gcc tgg ggg acc ttg ctg ttt tat ata ggt ggt cac ttg gta cga gat    96
   Ala Trp Gly Thr Leu Leu Phe Tyr Ile Gly Gly His Leu Val Arg Asp
               20                  25                  30
   aat gac cat cct gat cac tct agc cga gaa ctg tcc aag att ctg gca   144
   Asn Asp His Pro Asp His Ser Ser Arg Glu Leu Ser Lys Ile Leu Ala
           35                  40                  45
   aag ctt gaa cgc tta aaa caa cag aat gaa gac ttg agg cga atg gcc   192
   Lys Leu Glu Arg Leu Lys Gln Gln Asn Glu Asp Leu Arg Arg Met Ala
       50                  55                  60
   gaa tct ctc cgg ata cca gaa ggc cct att gat cag ggg cca gct ata   240
   Glu Ser Leu Arg Ile Pro Glu Gly Pro Ile Asp Gln Gly Pro Ala Ile
   65                  70                  75                  80
   gga aga gta cgc gtt tta gaa gag cag ctt gtt aag gcc aaa gaa cag   288
   Gly Arg Val Arg Val Leu Glu Glu Gln Leu Val Lys Ala Lys Glu Gln
                   85                  90                  95
   att gaa aat tac aag aaa cag acc aga aat ggt ctg ggg aag gat cat 336
   Ile Glu Asn Tyr Lys Lys Gln Thr Arg Asn Gly Leu Gly Lys Asp His
   100 105 110
   gaa atc ctg agg agg agg att gaa aat gga gct aaa gag ctc tgg ttt 384
   Glu Ile Leu Arg Arg Arg Ile Glu Asn Gly Ala Lys Glu Leu Trp Phe
   115 120 125
   ttc cta cag agt gaa ttg aag aaa tta aag aac tta gaa gga aat gaa 432
   Phe Leu Gln Ser Glu Leu Lys Lys Leu Lys Asn Leu Glu Gly Asn Glu
   130 135 140
   ctc caa aga cat gca gat gaa ttt ctt ttg gat tta gga cat cat gaa 480
   Leu Gln Arg His Ala Asp Glu Phe Leu Leu Asp Leu Gly His His Glu
   145 150 155 160
   agg tct ata atg acg gat cta tac tac ctc agt cag aca gat gga gca 528
   Arg Ser Ile Met Thr Asp Leu Tyr Tyr Leu Ser Gln Thr Asp Gly Ala
   165 170 175
   ggt gat tgg cgg gaa aaa gag gcc aaa gat ctg aca gaa ctg gtt cag 576
   Gly Asp Trp Arg Glu Lys Glu Ala Lys Asp Leu Thr Glu Leu Val Gln
   180 185 190
   cgg aga ata aca tat ctt cag aat ccc aag gac tgc agc aaa gcc aaa 624
   Arg Arg Ile Thr Tyr Leu Gln Asn Pro Lys Asp Cys Ser Lys Ala Lys
   195 200 205
   aag ctg gtg tgt aat atc aac aaa ggc tgt ggc tat ggc tgt cag ctc 672
   Lys Leu Val Cys Asn Ile Asn Lys Gly Cys Gly Tyr Gly Cys Gln Leu
   210 215 220
   cat cat gtg gtc tac tgc ttc atg att gca tat ggc acc cag cga aca 720
   His His Val Val Tyr Cys Phe Met Ile Ala Tyr Gly Thr Gln Arg Thr
   225 230 235 240
   ctc atc ttg gaa tct cag aat tgg cgc tat gct act ggt gga tgg gag 768
   Leu Ile Leu Glu Ser Gln Asn Trp Arg Tyr Ala Thr Gly Gly Trp Glu
   245 250 255
   act gta ttt agg cct gta agt gag aca tgc aca gac aga tct ggc atc 816
   Thr Val Phe Arg Pro Val Ser Glu Thr Cys Thr Asp Arg Ser Gly Ile
   260 265 270
   tcc act gga cac tgg tca ggt gaa gtg aag gac aaa aat gtt caa gtg 864
   Ser Thr Gly His Trp Ser Gly Glu Val Lys Asp Lys Asn Val Gln Val
   275 280 285
   gtc gag ctt ccc att gta gac agt ctt cat ccc cgt cct cca tat tta 912
   Val Glu Leu Pro Ile Val Asp Ser Leu His Pro Arg Pro Pro Tyr Leu
   290 295 300
   ccc ttg gct gta cca gaa gac ctc gca gat cga ctt gta cga gtg cat 960
   Pro Leu Ala Val Pro Glu Asp Leu Ala Asp Arg Leu Val Arg Val His
   305 310 315 320
   ggt gac cct gca gtg tgg tgg gtg tct cag ttt gtc aaa tac ttg atc 1008
   Gly Asp Pro Ala Val Trp Trp Val Ser Gln Phe Val Lys Tyr Leu Ile
   325 330 335
   cgc cca cag cct tgg cta gaa aaa gaa ata gaa gaa gcc acc aag aag 1056
   Arg Pro Gln Pro Trp Leu Glu Lys Glu Ile Glu Glu Ala Thr Lys Lys
   340 345 350
   ctt ggc ttc aaa cat cca gtt att gga gtc cat gtc aga cgc aca gac     1104
   Leu Gly Phe Lys His Pro Val Ile Gly Val His Val Arg Arg Thr Asp
           355                 360                 365
   aaa gtg gga aca gaa gct gcc ttc cat ccc att gaa gag tac atg gtg     1152
   Lys Val Gly Thr Glu Ala Ala Phe His Pro Ile Glu Glu Tyr Met Val
       370                 375                 380
   cat gtt gaa gaa cat ttt cag ctt ctt gca cgc aga atg caa gtg gac     1200
   His Val Glu Glu His Phe Gln Leu Leu Ala Arg Arg Met Gln Val Asp
   385                 390                 395                 400
   aaa aaa aga gtg tat ttg gcc aca gat gac cct tct tta tta aag gag     1248
   Lys Lys Arg Val Tyr Leu Ala Thr Asp Asp Pro Ser Leu Leu Lys Glu
                   405                 410                 415
   gca aaa aca aag tac ccc aat tat gaa ttt att agt gat aac tct att     1296
   Ala Lys Thr Lys Tyr Pro Asn Tyr Glu Phe Ile Ser Asp Asn Ser Ile
               420                 425                 430
   tcc tgg tca gct gga ctg cac aat cga tac aca gaa aat tca ctt cgt     1344
   Ser Trp Ser Ala Gly Leu His Asn Arg Tyr Thr Glu Asn Ser Leu Arg
           435                 440                 445
   gga gtg atc ctg gat ata cat ttt ctc tct cag gca gac ttc cta gtg     1392
   Gly Val Ile Leu Asp Ile His Phe Leu Ser Gln Ala Asp Phe Leu Val
       450                 455                 460
   tgt act ttt tca tcc cag gtc tgt cga gtt gct tat gaa att atg caa     1440
   Cys Thr Phe Ser Ser Gln Val Cys Arg Val Ala Tyr Glu Ile Met Gln
   465                 470                 475                 480
   aca cta cat cct gat gcc tct gca aac ttc cat tct tta gat gac atc     1488
   Thr Leu His Pro Asp Ala Ser Ala Asn Phe His Ser Leu Asp Asp Ile
                   485                 490                 495
   tac tat ttt ggg ggc cag aat gcc cac aat caa att gcc att tat gct     1536
   Tyr Tyr Phe Gly Gly Gln Asn Ala His Asn Gln Ile Ala Ile Tyr Ala
               500                 505                 510
   cac caa ccc cga act gca gat gaa att ccc atg gaa cct gga gat atc     1584
   His Gln Pro Arg Thr Ala Asp Glu Ile Pro Met Glu Pro Gly Asp Ile
           515                 520                 525
   att ggt gtg gct gga aat cat tgg gat ggc tat tct aaa ggt gtc aac     1632
   Ile Gly Val Ala Gly Asn His Trp Asp Gly Tyr Ser Lys Gly Val Asn
       530                 535                 540
   agg aaa ttg gga agg acg ggc cta tat ccc tcc tac aaa gtt cga gag     1680
   Arg Lys Leu Gly Arg Thr Gly Leu Tyr Pro Ser Tyr Lys Val Arg Glu
   545                 550                 555                 560
   aag ata gaa acg gtc aag tac ccc aca tat cct gag gct gag aaa taa     1728
   Lys Ile Glu Thr Val Lys Tyr Pro Thr Tyr Pro Glu Ala Glu Lys  *
                   565                 570                 575
<210> 8
   <211> 575
   <212> PRT
   <213> Homo sapians
<400> 8
   Met Arg Pro Trp Thr Gly Ser Trp Arg Trp Ile Met Leu Ile Leu Phe
   1               5                   10                  15
   Ala Trp Gly Thr Leu Leu Phe Tyr Ile Gly Gly His Leu Val Arg Asp
               20                  25                  30
   Asn Asp His Pro Asp His Ser Ser Arg Glu Leu Ser Lys Ile Leu Ala
           35                  40                  45
   Lys Leu Glu Arg Leu Lys Gln Gln Asn Glu Asp Leu Arg Arg Met Ala
       50                  55                  60
   Glu Ser Leu Arg Ile Pro Glu Gly Pro Ile Asp Gln Gly Pro Ala Ile
   65                  70                  75                  80
   Gly Arg Val Arg Val Leu Glu Glu Gln Leu Val Lys Ala Lys Glu Gln
                   85                  90                  95
   Ile Glu Asn Tyr Lys Lys Gln Thr Arg Asn Gly Leu Gly Lys Asp His
               100                 105                 110
   Glu Ile Leu Arg Arg Arg Ile Glu Asn Gly Ala Lys Glu Leu Trp Phe
           115                 120                 125
   Phe Leu Gln Ser Glu Leu Lys Lys Leu Lys Asn Leu Glu Gly Asn Glu
       130                 135                 140
   Leu Gln Arg His Ala Asp Glu Phe Leu Leu Asp Leu Gly His His Glu
   145                 150                 155                 160
   Arg Ser Ile Met Thr Asp Leu Tyr Tyr Leu Ser Gln Thr Asp Gly Ala
                   165                 170                 175
   Gly Asp Trp Arg Glu Lys Glu Ala Lys Asp Leu Thr Glu Leu Val Gln
               180                 185                 190
   Arg Arg Ile Thr Tyr Leu Gln Asn Pro Lys Asp Cys Ser Lys Ala Lys
           195                 200                 205
   Lys Leu Val Cys Asn Ile Asn Lys Gly Cys Gly Tyr Gly Cys Gln Leu
       210                 215                 220
   His His Val Val Tyr Cys Phe Met Ile Ala Tyr Gly Thr Gln Arg Thr
   225                 230                 235                 240
   Leu Ile Leu Glu Ser Gln Asn Trp Arg Tyr Ala Thr Gly Gly Trp Glu
                   245                 250                 255
   Thr Val Phe Arg Pro Val Ser Glu Thr Cys Thr Asp Arg Ser Gly Ile
               260                 265                 270
   Ser Thr Gly His Trp Ser Gly Glu Val Lys Asp Lys Asn Val Gln Val
           275                 280                 285
   Val Glu Leu Pro Ile Val Asp Ser Leu His Pro Arg Pro Pro Tyr Leu
       290                 295                 300
   Pro Leu Ala Val Pro Glu Asp Leu Ala Asp Arg Leu Val Arg Val His
   305                 310                 315                 320
   Gly Asp Pro Ala Val Trp Trp Val Ser Gln Phe Val Lys Tyr Leu Ile
                   325                 330                 335
   Arg Pro Gln Pro Trp Leu Glu Lys Glu Ile Glu Glu Ala Thr Lys Lys
               340                 345                 350
   Leu Gly Phe Lys His Pro Val Ile Gly Val His Val Arg Arg Thr Asp
           355                 360                 365
   Lys Val Gly Thr Glu Ala Ala Phe His Pro Ile Glu Glu Tyr Met Val
       370                 375                 380
   His Val Glu Glu His Phe Gln Leu Leu Ala Arg Arg Met Gln Val Asp
   385                 390                 395                 400
   Lys Lys Arg Val Tyr Leu Ala Thr Asp Asp Pro Ser Leu Leu Lys Glu
                   405                 410                 415
   Ala Lys Thr Lys Tyr Pro Asn Tyr Glu Phe Ile Ser Asp Asn Ser Ile
               420                 425                 430
   Ser Trp Ser Ala Gly Leu His Asn Arg Tyr Thr Glu Asn Ser Leu Arg
           435                 440                 445
   Gly Val Ile Leu Asp Ile His Phe Leu Ser Gln Ala Asp Phe Leu Val
       450                 455                 460
   Cys Thr Phe Ser Ser Gln Val Cys Arg Val Ala Tyr Glu Ile Met Gln
   465                 470                 475                 480
   Thr Leu His Pro Asp Ala Ser Ala Asn Phe His Ser Leu Asp Asp Ile
                   485                 490                 495
   Tyr Tyr Phe Gly Gly Gln Asn Ala His Asn Gln Ile Ala Ile Tyr Ala
               500                 505                 510
   His Gln Pro Arg Thr Ala Asp Glu Ile Pro Met Glu Pro Gly Asp Ile
           515                 520                 525
   Ile Gly Val Ala Gly Asn His Trp Asp Gly Tyr Ser Lys Gly Val Asn
       530                 535                 540
   Arg Lys Leu Gly Arg Thr Gly Leu Tyr Pro Ser Tyr Lys Val Arg Glu
   545                 550                 555                 560
   Lys Ile Glu Thr Val Lys Tyr Pro Thr Tyr Pro Glu Ala Glu Lys
                   565                 570                 575
<210> 9
   <211> 1728
   <212> DNA
   <213> Rattus norvegicus
<220>
   <221> CDS
   <222> (1)...(1728)
   <223> alpha-1,6-fucosyltransferase (rFuT8)
<400> 9
   atg cgg gca tgg act ggt tcc tgg cgt tgg att atg ctc att ctt ttt    48
   Met Arg Ala Trp Thr Gly Ser Trp Arg Trp Ile Met Leu Ile Leu Phe
    1               5                  10                  15
   gcc tgg ggg acc ttg ttg ttt tat ata ggt ggt cat ttg gtt cga gat    96
   Ala Trp Gly Thr Leu Leu Phe Tyr Ile Gly Gly His Leu Val Arg Asp
               20                  25                  30
   aat gac cac cct gat cac tct agc aga gaa ctc tcc aag att ctt gca   144
   Asn Asp His Pro Asp His Ser Ser Arg Glu Leu Ser Lys Ile Leu Ala
           35                  40                  45
   aag ctt gaa cgc tta aaa caa caa aat gaa gac ttg agg cga atg gct   192
   Lys Leu Glu Arg Leu Lys Gln Gln Asn Glu Asp Leu Arg Arg Met Ala
       50                  55                  60
   gag tct cta cga ata cca gaa ggc ccc att gac cag ggg acg gct acg   240
   Glu Ser Leu Arg Ile Pro Glu Gly Pro Ile Asp Gln Gly Thr Ala Thr
    65                  70                  75                  80
   gga aga gtc cgt gtt tta gaa gaa cag ctt gtt aag gcc aaa gaa cag   288
   Gly Arg Val Arg Val Leu Glu Glu Gln Leu Val Lys Ala Lys Glu Gln
                   85                  90                  95
   att gaa aat tac aag aaa caa gcc aga aat ggt ctg ggg aag gat cat   336
   Ile Glu Asn Tyr Lys Lys Gln Ala Arg Asn Gly Leu Gly Lys Asp His
               100                 105                 110
   gaa ctc tta agg agg agg att gaa aat gga gct aaa gag ctc tgg ttt   384
   Glu Leu Leu Arg Arg Arg Ile Glu Asn Gly Ala Lys Glu Leu Trp Phe
           115                 120                 125
   ttt cta caa agt gaa ctg aag aaa tta aag cat cta gaa gga aat gaa   432
   Phe Leu Gln Ser Glu Leu Lys Lys Leu Lys His Leu Glu Gly Asn Glu
       130                 135                 140
   ctc caa aga cat gca gat gaa att ctt ttg gat tta gga cac cat gaa   480
   Leu Gln Arg His Ala Asp Glu Ile Leu Leu Asp Leu Gly His His Glu
   145                 150                 155                 160
   agg tct atc atg acg gat cta tac tac ctc agt caa aca gat gga gca   528
   Arg Ser Ile Met Thr Asp Leu Tyr Tyr Leu Ser Gln Thr Asp Gly Ala
                   165                 170                 175
   ggg gat tgg cgt gaa aaa gag gcc aaa gat ctg aca gag ctg gtc cag   576
   Gly Asp Trp Arg Glu Lys Glu Ala Lys Asp Leu Thr Glu Leu Val Gln
               180                 185                 190
   cgg aga ata act tat ctc cag aat ccc aag gac tgc agc aaa gcc agg    624
   Arg Arg Ile Thr Tyr Leu Gln Asn Pro Lys Asp Cys Ser Lys Ala Arg
           195                 200                 205
   aag ctg gtg tgt aac atc aat aag ggc tgt ggc tat ggt tgc caa ctc    672
   Lys Leu Val Cys Asn Ile Asn Lys Gly Cys Gly Tyr Gly Cys Gln Leu
       210                 215                 220
   cat cac gtg gtc tac tgt ttc atg att gct tat ggc acc cag cga aca    720
   His His Val Val Tyr Cys Phe Met Ile Ala Tyr Gly Thr Gln Arg Thr
   225                 230                 235                 240
   ctc atc ttg gaa tct cag aat tgg cgc tat gct act ggt gga tgg gag    768
   Leu Ile Leu Glu Ser Gln Asn Trp Arg Tyr Ala Thr Gly Gly Trp Glu
                   245                 250                 255
   act gtg ttt aga cct gta agt gag aca tgc aca gac aga tct ggc ctc    816
   Thr Val Phe Arg Pro Val Ser Glu Thr Cys Thr Asp Arg Ser Gly Leu
               260                 265                 270
   tcc act gga cac tgg tca ggt gaa gtg aat gac aaa aat att caa gtg    864
   Ser Thr Gly His Trp Ser Gly Glu Val Asn Asp Lys Asn Ile Gln Val
           275                 280                 285
   gtg gag ctc ccc att gta gac agc ctc cat cct cgg cct cct tac tta    912
   Val Glu Leu Pro Ile Val Asp Ser Leu His Pro Arg Pro Pro Tyr Leu
       290                 295                 300
   cca ctg gct gtt cca gaa gac ctt gca gat cga ctc gta aga gtc cat    960
   Pro Leu Ala Val Pro Glu Asp Leu Ala Asp Arg Leu Val Arg Val His
   305                 310                 315                 320
   ggt gat cct gca gtg tgg tgg gtg tcc cag ttc gtc aaa tat ttg att    1008
   Gly Asp Pro Ala Val Trp Trp Val Ser Gln Phe Val Lys Tyr Leu Ile
                   325                 330                 335
   cgt cca caa cct tgg cta gaa aag gaa ata gaa gaa gcc acc aag aag    1056
   Arg Pro Gln Pro Trp Leu Glu Lys Glu Ile Glu Glu Ala Thr Lys Lys
               340                 345                 350
   ctt ggc ttc aaa cat cca gtc att gga gtc cat gtc aga cgc aca gac    1104
   Leu Gly Phe Lys His Pro Val Ile Gly Val His Val Arg Arg Thr Asp
           355                 360                 365
   aaa gtg gga aca gag gca gcc ttc cat ccc atc gaa gag tac atg gta    1152
   Lys Val Gly Thr Glu Ala Ala Phe His Pro Ile Glu Glu Tyr Met Val
       370                 375                 380
   cat gtt gaa gaa cat ttt cag ctt ctc gca cgc aga atg caa gtg gat    1200
   His Val Glu Glu His Phe Gln Leu Leu Ala Arg Arg Met Gln Val Asp
   385                 390                 395                 400
   aaa aaa aga gta tat ctg gct acc gat gac cct gct ttg tta aag gag    1248
   Lys Lys Arg Val Tyr Leu Ala Thr Asp Asp Pro Ala Leu Leu Lys Glu
                   405                 410                 415
   gca aag aca aag tac tcc aat tat gaa ttt att agt gat aac tct att    1296
   Ala Lys Thr Lys Tyr Ser Asn Tyr Glu Phe Ile Ser Asp Asn Ser Ile
               420                 425                 430
   tct tgg tca gct gga tta cac aat cgg tac aca gaa aat tca ctt cgg    1344
   Ser Trp Ser Ala Gly Leu His Asn Arg Tyr Thr Glu Asn Ser Leu Arg
           435                 440                 445
   ggc gtg atc ctg gat ata cac ttt ctc tct cag gct gac ttc cta gtg    1392
   Gly Val Ile Leu Asp Ile His Phe Leu Ser Gln Ala Asp Phe Leu Val
       450                 455                 460
   tgt act ttt tca tcc cag gtc tgt cgg gtt gct tat gaa atc atg caa    1440
   Cys Thr Phe Ser Ser Gln Val Cys Arg Val Ala Tyr Glu Ile Met Gln
   465                 470                 475                 480
   acc ctg cat cct gat gcc tct gca aac ttc cac tct tta gat gac atc    1488
   Thr Leu His Pro Asp Ala Ser Ala Asn Phe His Ser Leu Asp Asp Ile
                   485                 490                 495
   tac tat ttt gga ggc caa aat gcc cac aac cag att gcc gtt tat cct    1536
   Tyr Tyr Phe Gly Gly Gln Asn Ala His Asn Gln Ile Ala Val Tyr Pro
               500                 505                 510
   cac aaa cct cga act gat gag gaa att cca atg gaa cct gga gat atc    1584
   His Lys Pro Arg Thr Asp Glu Glu Ile Pro Met Glu Pro Gly Asp Ile
           515                 520                 525
   att ggt gtg gct gga aac cat tgg gat ggt tat tct aaa ggt gtc aac    1632
   Ile Gly Val Ala Gly Asn His Trp Asp Gly Tyr Ser Lys Gly Val Asn
       530                 535                 540
   aga aaa ctt gga aaa aca ggc tta tat ccc tcc tac aaa gtc cga gag    1680
   Arg Lys Leu Gly Lys Thr Gly Leu Tyr Pro Ser Tyr Lys Val Arg Glu
   545                 550                 555                 560
   aag ata gaa aca gtc aag tat ccc aca tat cct gaa gct gaa aaa tag    1728
   Lys Ile Glu Thr Val Lys Tyr Pro Thr Tyr Pro Glu Ala Glu Lys  *
                   565                 570                 575
<210> 10
   <211> 575
   <212> PRT
   <213> Rattus norvegicus
<400> 10
   Met Arg Ala Trp Thr Gly Ser Trp Arg Trp Ile Met Leu Ile Leu Phe
    1               5                  10                  15
   Ala Trp Gly Thr Leu Leu Phe Tyr Ile Gly Gly His Leu Val Arg Asp
               20                  25                  30
   Asn Asp His Pro Asp His Ser Ser Arg Glu Leu Ser Lys Ile Leu Ala
           35                  40                  45
   Lys Leu Glu Arg Leu Lys Gln Gln Asn Glu Asp Leu Arg Arg Met Ala
       50                  55                  60
   Glu Ser Leu Arg Ile Pro Glu Gly Pro Ile Asp Gln Gly Thr Ala Thr
   65                  70                  75                  80
   Gly Arg Val Arg Val Leu Glu Glu Gln Leu Val Lys Ala Lys Glu Gln
                   85                  90                  95
   Ile Glu Asn Tyr Lys Lys Gln Ala Arg Asn Gly Leu Gly Lys Asp His
               100                 105                 110
   Glu Leu Leu Arg Arg Arg Ile Glu Asn Gly Ala Lys Glu Leu Trp Phe
           115                 120                 125
   Phe Leu Gln Ser Glu Leu Lys Lys Leu Lys His Leu Glu Gly Asn Glu
       130                 135                 140
   Leu Gln Arg His Ala Asp Glu Ile Leu Leu Asp Leu Gly His His Glu
   145                 150                 155                 160
   Arg Ser Ile Met Thr Asp Leu Tyr Tyr Leu Ser Gln Thr Asp Gly Ala
                   165                 170                 175
   Gly Asp Trp Arg Glu Lys Glu Ala Lys Asp Leu Thr Glu Leu Val Gln
               180                 185                 190
   Arg Arg Ile Thr Tyr Leu Gln Asn Pro Lys Asp Cys Ser Lys Ala Arg
           195                 200                 205
   Lys Leu Val Cys Asn Ile Asn Lys Gly Cys Gly Tyr Gly Cys Gln Leu
       210                 215                 220
   His His Val Val Tyr Cys Phe Met Ile Ala Tyr Gly Thr Gln Arg Thr
   225                 230                 235                 240
   Leu Ile Leu Glu Ser Gln Asn Trp Arg Tyr Ala Thr Gly Gly Trp Glu
                   245                 250                 255
   Thr Val Phe Arg Pro Val Ser Glu Thr Cys Thr Asp Arg Ser Gly Leu
               260                 265                 270
   Ser Thr Gly His Trp Ser Gly Glu Val Asn Asp Lys Asn Ile Gln Val
           275                 280                 285
   Val Glu Leu Pro Ile Val Asp Ser Leu His Pro Arg Pro Pro Tyr Leu
       290                 295                 300
   Pro Leu Ala Val Pro Glu Asp Leu Ala Asp Arg Leu Val Arg Val His
   305                 310                 315                 320
   Gly Asp Pro Ala Val Trp Trp Val Ser Gln Phe Val Lys Tyr Leu Ile
                   325                 330                 335
   Arg Pro Gln Pro Trp Leu Glu Lys Glu Ile Glu Glu Ala Thr Lys Lys
               340                 345                 350
   Leu Gly Phe Lys His Pro Val Ile Gly Val His Val Arg Arg Thr Asp
           355                 360                 365
   Lys Val Gly Thr Glu Ala Ala Phe His Pro Ile Glu Glu Tyr Met Val
       370                 375                 380
   His Val Glu Glu His Phe Gln Leu Leu Ala Arg Arg Met Gln Val Asp
   385                 390                 395                 400
   Lys Lys Arg Val Tyr Leu Ala Thr Asp Asp Pro Ala Leu Leu Lys Glu
                   405                 410                 415
   Ala Lys Thr Lys Tyr Ser Asn Tyr Glu Phe Ile Ser Asp Asn Ser Ile
               420                 425                 430
   Ser Trp Ser Ala Gly Leu His Asn Arg Tyr Thr Glu Asn Ser Leu Arg
           435                 440                 445
   Gly Val Ile Leu Asp Ile His Phe Leu Ser Gln Ala Asp Phe Leu Val
       450                 455                 460
   Cys Thr Phe Ser Ser Gln Val Cys Arg Val Ala Tyr Glu Ile Met Gln
   465                 470                 475                 480
   Thr Leu His Pro Asp Ala Ser Ala Asn Phe His Ser Leu Asp Asp Ile
                   485                 490                 495
   Tyr Tyr Phe Gly Gly Gln Asn Ala His Asn Gln Ile Ala Val Tyr Pro
               500                 505                 510
   His Lys Pro Arg Thr Asp Glu Glu Ile Pro Met Glu Pro Gly Asp Ile
           515                 520                 525
   Ile Gly Val Ala Gly Asn His Trp Asp Gly Tyr Ser Lys Gly Val Asn
       530                 535                 540
   Arg Lys Leu Gly Lys Thr Gly Leu Tyr Pro Ser Tyr Lys Val Arg Glu
   545                 550                 555                 560
   Lys Ile Glu Thr Val Lys Tyr Pro Thr Tyr Pro Glu Ala Glu Lys
                   565                 570                 575
<210> 11
   <211> 1728
   <212> DNA
   <213> Mus musculus
<220>
   <221> CDS
   <222> (1)...(1728)
   <223> alpha-1,6-fucosyltransferase (mFuT8)
<400> 11
   atg cgg gca tgg act ggt tcc tgg cgt tgg att atg ctc att ctt ttt    48
   Met Arg Ala Trp Thr Gly Ser Trp Arg Trp Ile Met Leu Ile Leu Phe
    1               5                   10                  15
   gcc tgg ggg acc ttg tta ttt tat ata ggt ggt cat ttg gtt cga gat     96
   Ala Trp Gly Thr Leu Leu Phe Tyr Ile Gly Gly His Leu Val Arg Asp
               20                  25                  30
   aat gac cac cct gat cac tcc agc aga gaa ctc tcc aag att ctt gca    144
   Asn Asp His Pro Asp His Ser Ser Arg Glu Leu Ser Lys Ile Leu Ala
           35                  40                  45
   aag ctt gaa cgc tta aaa cag caa aat gaa gac ttg agg cga atg gct    192
   Lys Leu Glu Arg Leu Lys Gln Gln Asn Glu Asp Leu Arg Arg Met Ala
       50                  55                  60
   gag tct ctc cga ata cca gaa ggc ccc att gac cag ggg aca gct aca    240
   Glu Ser Leu Arg Ile Pro Glu Gly Pro Ile Asp Gln Gly Thr Ala Thr
   65                  70                  75                  80
   gga aga gtc cgt gtt tta gaa gaa cag ctt gtt aag gcc aaa gaa cag    288
   Gly Arg Val Arg Val Leu Glu Glu Gln Leu Val Lys Ala Lys Glu Gln
                   85                  90                  95
   att gaa aat tac aag aaa caa gct aga aat ggt ctg ggg aag gat cat    336
   Ile Glu Asn Tyr Lys Lys Gln Ala Arg Asn Gly Leu Gly Lys Asp His
               100                 105                 110
   gaa atc tta aga agg agg att gaa aat gga gct aaa gag ctc tgg ttt    384
   Glu Ile Leu Arg Arg Arg Ile Glu Asn Gly Ala Lys Glu Leu Trp Phe
           115                 120                 125
   ttt cta caa agc gaa ctg aag aaa tta aag cat tta gaa gga aat gaa    432
   Phe Leu Gln Ser Glu Leu Lys Lys Leu Lys His Leu Glu Gly Asn Glu
       130                 135                 140
   ctc caa aga cat gca gat gaa att ctt ttg gat tta gga cac cat gaa    480
   Leu Gln Arg His Ala Asp Glu Ile Leu Leu Asp Leu Gly His His Glu
   145                 150                 155                 160
   agg tct atc atg aca gat cta tac tac ctc agt caa aca gat gga gca    528
   Arg Ser Ile Met Thr Asp Leu Tyr Tyr Leu Ser Gln Thr Asp Gly Ala
                   165                 170                 175
   ggg gat tgg cgt gaa aaa gag gcc aaa gat ctg aca gag ctg gtc cag    576
   Gly Asp Trp Arg Glu Lys Glu Ala Lys Asp Leu Thr Glu Leu Val Gln
               180                 185                 190
   cgg aga ata aca tat ctc cag aat cct aag gac tgc agc aaa gcc agg    624
   Arg Arg Ile Thr Tyr Leu Gln Asn Pro Lys Asp Cys Ser Lys Ala Arg
           195                 200                 205
   aag ctg gtg tgt aac atc aat aaa ggc tgt ggc tat ggt tgt caa ctc    672
   Lys Leu Val Cys Asn Ile Asn Lys Gly Cys Gly Tyr Gly Cys Gln Leu
       210                 215                 220
   cat cac gtg gtc tac tgt ttc atg att gct tat ggc acc cag cga aca    720
   His His Val Val Tyr Cys Phe Met Ile Ala Tyr Gly Thr Gln Arg Thr
   225                 230                 235                 240
   ctc atc ttg gaa tct cag aat tgg cgc tat gct act ggt gga tgg gag    768
   Leu Ile Leu Glu Ser Gln Asn Trp Arg Tyr Ala Thr Gly Gly Trp Glu
                   245                 250                 255
   act gtg ttt aga cct gta agt gag aca tgt aca gac aga tct ggc ctc    816
   Thr Val Phe Arg Pro Val Ser Glu Thr Cys Thr Asp Arg Ser Gly Leu
               260                 265                 270
   tcc act gga cac tgg tca ggt gaa gta aat gac aaa aac att caa gtg    864
   Ser Thr Gly His Trp Ser Gly Glu Val Asn Asp Lys Asn Ile Gln Val
           275                 280                 285
   gtc gag ctc ccc att gta gac agc ctc cat cct cgg cct cct tac tta      912
   Val Glu Leu Pro Ile Val Asp Ser Leu His Pro Arg Pro Pro Tyr Leu
       290                 295                 300
   cca ctg gct gtt cca gaa gac ctt gca gac cga ctc cta aga gtc cat      960
   Pro Leu Ala Val Pro Glu Asp Leu Ala Asp Arg Leu Leu Arg Val His
   305                 310                 315                 320
   ggt gac cct gca gtg tgg tgg gtg tcc cag ttt gtc aaa tac ttg att     1008
   Gly Asp Pro Ala Val Trp Trp Val Ser Gln Phe Val Lys Tyr Leu Ile
                   325                 330                 335
   cgt cca caa cct tgg ctg gaa aag gaa ata gaa gaa gcc acc aag aag     1056
   Arg Pro Gln Pro Trp Leu Glu Lys Glu Ile Glu Glu Ala Thr Lys Lys
               340                 345                 350
   ctt ggc ttc aaa cat cca gtt att gga gtc cat gtc aga cgc aca gac     1104
   Leu Gly Phe Lys His Pro Val Ile Gly Val His Val Arg Arg Thr Asp
           355                 360                 365
   aaa gtg gga aca gaa gca gcc ttc cac ccc atc gag gag tac atg gta     1152
   Lys Val Gly Thr Glu Ala Ala Phe His Pro Ile Glu Glu Tyr Met Val
       370                 375                 380
   cac gtt gaa gaa cat ttt cag ctt ctc gca cgc aga atg caa gtg gat     1200
   His Val Glu Glu His Phe Gln Leu Leu Ala Arg Arg Met Gln Val Asp
   385                 390                 395                 400
   aaa aaa aga gta tat ctg gct act gat gat cct act ttg tta aag gag     1248
   Lys Lys Arg Val Tyr Leu Ala Thr Asp Asp Pro Thr Leu Leu Lys Glu
                   405                 410                 415
   gca aag aca aag tac tcc aat tat gaa ttt att agt gat aac tct att     1296
   Ala Lys Thr Lys Tyr Ser Asn Tyr Glu Phe Ile Ser Asp Asn Ser Ile
               420                 425                 430
   tct tgg tca gct gga cta cac aat cgg tac aca gaa aat tca ctt cgg     1344
   Ser Trp Ser Ala Gly Leu His Asn Arg Tyr Thr Glu Asn Ser Leu Arg
           435                 440                 445
   ggt gtg atc ctg gat ata cac ttt ctc tca cag gct gac ttt cta gtg     1392
   Gly Val Ile Leu Asp Ile His Phe Leu Ser Gln Ala Asp Phe Leu Val
       450                 455                 460
   tgt act ttt tca tcc cag gtc tgt cgg gtt gct tat gaa atc atg caa     1440
   Cys Thr Phe Ser Ser Gln Val Cys Arg Val Ala Tyr Glu Ile Met Gln
   465                 470                 475                 480
   acc ctg cat cct gat gcc tct gcg aac ttc cat tct ttg gat gac atc     1488
   Thr Leu His Pro Asp Ala Ser Ala Asn Phe His Ser Leu Asp Asp Ile
                   485                 490                 495
   tac tat ttt gga ggc caa aat gcc cac aat cag att gct gtt tat cct     1536
   Tyr Tyr Phe Gly Gly Gln Asn Ala His Asn Gln Ile Ala Val Tyr Pro
               500                 505                 510
   cac aaa cct cga act gaa gag gaa att cca atg gaa cct gga gat atc     1584
   His Lys Pro Arg Thr Glu Glu Glu Ile Pro Met Glu Pro Gly Asp Ile
           515                 520                 525
   att ggt gtg gct gga aac cat tgg gat ggt tat tct aaa ggt atc aac     1632
   Ile Gly Val Ala Gly Asn His Trp Asp Gly Tyr Ser Lys Gly Ile Asn
       530                 535                 540
   aga aaa ctt gga aaa aca ggc tta tat ccc tcc tac aaa gtc cga gag    1680
   Arg Lys Leu Gly Lys Thr Gly Leu Tyr Pro Ser Tyr Lys Val Arg Glu
   545                 550                 555                 560
   aag ata gaa aca gtc aag tat ccc aca tat cct gaa gct gaa aaa tag    1728
   Lys Ile Glu Thr Val Lys Tyr Pro Thr Tyr Pro Glu Ala Glu Lys  *
                   565                 570                 575
<210> 12
   <211> 575
   <212> PRT
   <213> Mus musculus
<400> 12
   Met Arg Ala Trp Thr Gly Ser Trp Arg Trp Ile Met Leu Ile Leu Phe
    1               5                  10                  15
   Ala Trp Gly Thr Leu Leu Phe Tyr Ile Gly Gly His Leu Val Arg Asp
               20                  25                  30
   Asn Asp His Pro Asp His Ser Ser Arg Glu Leu Ser Lys Ile Leu Ala
           35                  40                  45
   Lys Leu Glu Arg Leu Lys Gln Gln Asn Glu Asp Leu Arg Arg Met Ala
       50                  55                  60
   Glu Ser Leu Arg Ile Pro Glu Gly Pro Ile Asp Gln Gly Thr Ala Thr
   65                  70                  75                  80
   Gly Arg Val Arg Val Leu Glu Glu Gln Leu Val Lys Ala Lys Glu Gln
                   85                  90                  95
   Ile Glu Asn Tyr Lys Lys Gln Ala Arg Asn Gly Leu Gly Lys Asp His
               100                 105                 110
   Glu Ile Leu Arg Arg Arg Ile Glu Asn Gly Ala Lys Glu Leu Trp Phe
           115                 120                 125
   Phe Leu Gln Ser Glu Leu Lys Lys Leu Lys His Leu Glu Gly Asn Glu
       130                 135                 140
   Leu Gln Arg His Ala Asp Glu Ile Leu Leu Asp Leu Gly His His Glu
   145                 150                 155                 160
   Arg Ser Ile Met Thr Asp Leu Tyr Tyr Leu Ser Gln Thr Asp Gly Ala
                   165                 170                 175
   Gly Asp Trp Arg Glu Lys Glu Ala Lys Asp Leu Thr Glu Leu Val Gln
               180                 185                 190
   Arg Arg Ile Thr Tyr Leu Gln Asn Pro Lys Asp Cys Ser Lys Ala Arg
           195                 200                 205
   Lys Leu Val Cys Asn Ile Asn Lys Gly Cys Gly Tyr Gly Cys Gln Leu
       210                 215                 220
   His His Val Val Tyr Cys Phe Met Ile Ala Tyr Gly Thr Gln Arg Thr
   225                 230                 235                 240
   Leu Ile Leu Glu Ser Gln Asn Trp Arg Tyr Ala Thr Gly Gly Trp Glu
                   245                 250                 255
   Thr Val Phe Arg Pro Val Ser Glu Thr Cys Thr Asp Arg Ser Gly Leu
               260                 265                 270
   Ser Thr Gly His Trp Ser Gly Glu Val Asn Asp Lys Asn Ile Gln Val
           275                 280                 285
   Val Glu Leu Pro Ile Val Asp Ser Leu His Pro Arg Pro Pro Tyr Leu
       290                 295                 300
   Pro Leu Ala Val Pro Glu Asp Leu Ala Asp Arg Leu Leu Arg Val His
   305                 310                 315                 320
   Gly Asp Pro Ala Val Trp Trp Val Ser Gln Phe Val Lys Tyr Leu Ile
                   325                 330                 335
   Arg Pro Gln Pro Trp Leu Glu Lys Glu Ile Glu Glu Ala Thr Lys Lys
               340                 345                 350
   Leu Gly Phe Lys His Pro Val Ile Gly Val His Val Arg Arg Thr Asp
           355                 360                 365
   Lys Val Gly Thr Glu Ala Ala Phe His Pro Ile Glu Glu Tyr Met Val
       370                 375                 380
   His Val Glu Glu His Phe Gln Leu Leu Ala Arg Arg Met Gln Val Asp
   385                 390                 395                 400
   Lys Lys Arg Val Tyr Leu Ala Thr Asp Asp Pro Thr Leu Leu Lys Glu
                   405                 410                 415
   Ala Lys Thr Lys Tyr Ser Asn Tyr Glu Phe Ile Ser Asp Asn Ser Ile
               420                 425                 430
   Ser Trp Ser Ala Gly Leu His Asn Arg Tyr Thr Glu Asn Ser Leu Arg
           435                 440                 445
   Gly Val Ile Leu Asp Ile His Phe Leu Ser Gln Ala Asp Phe Leu Val
       450                 455                 460
   Cys Thr Phe Ser Ser Gln Val Cys Arg Val Ala Tyr Glu Ile Met Gln
   465                 470                 475                 480
   Thr Leu His Pro Asp Ala Ser Ala Asn Phe His Ser Leu Asp Asp Ile
                   485                 490                 495
   Tyr Tyr Phe Gly Gly Gln Asn Ala His Asn Gln Ile Ala Val Tyr Pro
               500                 505                 510
   His Lys Pro Arg Thr Glu Glu Glu Ile Pro Met Glu Pro Gly Asp Ile
           515                 520                 525
   Ile Gly Val Ala Gly Asn His Trp Asp Gly Tyr Ser Lys Gly Ile Asn
       530                 535                 540
   Arg Lys Leu Gly Lys Thr Gly Leu Tyr Pro Ser Tyr Lys Val Arg Glu
   545                 550                 555                 560
   Lys Ile Glu Thr Val Lys Tyr Pro Thr Tyr Pro Glu Ala Glu Lys
                   565                 570                 575
<210> 13
   <211> 1728
   <212> DNA
   <213> Sus scrofa
<220>
   <221> CDS
   <222> (1)...(1728)
   <223> alpha-1,6-fucosyltransferase (pFuT8)
<400> 13
   atg cgg cca tgg act ggt tcg tgg cgt tgg att atg ctc att ctt ttt    48
   Met Arg Pro Trp Thr Gly Ser Trp Arg Trp Ile Met Leu Ile Leu Phe
    1               5                  10                  15
   gcc tgg ggg acc ttg cta ttt tac ata ggt ggt cac ttg gta cga gat    96
   Ala Trp Gly Thr Leu Leu Phe Tyr Ile Gly Gly His Leu Val Arg Asp
               20                  25                  30
   aat gac cac tct gat cac tct agc cga gaa ctg tcc aag att ttg gca   144
   Asn Asp His Ser Asp His Ser Ser Arg Glu Leu Ser Lys Ile Leu Ala
           35                  40                  45
   aag ctg gaa cgc tta aaa caa caa aat gaa gac ttg agg aga atg gct   192
   Lys Leu Glu Arg Leu Lys Gln Gln Asn Glu Asp Leu Arg Arg Met Ala
       50                  55                  60
   gaa tct ctc cga ata cca gaa ggc ccc att gat cag ggg cca gct tca   240
   Glu Ser Leu Arg Ile Pro Glu Gly Pro Ile Asp Gln Gly Pro Ala Ser
   65                  70                  75                  80
   gga aga gtt cgt gct tta gaa gag caa ttt atg aag gcc aaa gaa cag   288
   Gly Arg Val Arg Ala Leu Glu Glu Gln Phe Met Lys Ala Lys Glu Gln
                   85                  90                  95
   att gaa aat tat aag aaa caa act aaa aat ggt cca ggg aag gat cat   336
   Ile Glu Asn Tyr Lys Lys Gln Thr Lys Asn Gly Pro Gly Lys Asp His
               100                 105                 110
   gaa atc cta agg agg agg att gaa aat gga gct aaa gag ctc tgg ttt    384
   Glu Ile Leu Arg Arg Arg Ile Glu Asn Gly Ala Lys Glu Leu Trp Phe
           115                 120                 125
   ttt cta caa agt gag ttg aag aaa tta aag aat tta gaa gga aat gaa    432
   Phe Leu Gln Ser Glu Leu Lys Lys Leu Lys Asn Leu Glu Gly Asn Glu
       130                 135                 140
   ctc caa aga cat gca gat gaa ttt cta tca gat ttg gga cat cat gaa    480
   Leu Gln Arg His Ala Asp Glu Phe Leu Ser Asp Leu Gly His His Glu
   145                 150                 155                 160
   agg tct ata atg acg gat cta tac tac ctc agt caa aca gat ggg gca    528
   Arg Ser Ile Met Thr Asp Leu Tyr Tyr Leu Ser Gln Thr Asp Gly Ala
                   165                 170                 175
   ggt gat tgg cgt gaa aag gag gcc aaa gat ctg aca gag ctg gtc cag    576
   Gly Asp Trp Arg Glu Lys Glu Ala Lys Asp Leu Thr Glu Leu Val Gln
               180                 185                 190
   cgg aga ata aca tat ctt cag aat ccc aag gac tgc agc aaa gcc aag    624
   Arg Arg Ile Thr Tyr Leu Gln Asn Pro Lys Asp Cys Ser Lys Ala Lys
           195                 200                 205
   aag cta gtg tgt aat atc aac aaa ggc tgt ggc tat ggc tgt cag ctc    672
   Lys Leu Val Cys Asn Ile Asn Lys Gly Cys Gly Tyr Gly Cys Gln Leu
       210                 215                 220
   cat cat gta gtg tac tgc ttt atg att gca tat ggc acc cag cga aca    720
   His His Val Val Tyr Cys Phe Met Ile Ala Tyr Gly Thr Gln Arg Thr
   225                 230                 235                 240
   ctc gcc ttg gaa tct cac aat tgg cgc tac gct act ggg gga tgg gaa    768
   Leu Ala Leu Glu Ser His Asn Trp Arg Tyr Ala Thr Gly Gly Trp Glu
                   245                 250                 255
   act gtg ttt aga cct gta agt gag acg tgc aca gac aga tct ggc agc    816
   Thr Val Phe Arg Pro Val Ser Glu Thr Cys Thr Asp Arg Ser Gly Ser
               260                 265                 270
   tcc act gga cat tgg tca ggt gaa gta aag gac aaa aat gtt cag gtg    864
   Ser Thr Gly His Trp Ser Gly Glu Val Lys Asp Lys Asn Val Gln Val
           275                 280                 285
   gtt gag ctc ccc att gta gac agt gtt cat cct cgt cct cca tat tta    912
   Val Glu Leu Pro Ile Val Asp Ser Val His Pro Arg Pro Pro Tyr Leu
       290                 295                 300
   ccc ctg gct gtc cca gaa gac ctt gca gat cga ctt gta cga gtc cat    960
   Pro Leu Ala Val Pro Glu Asp Leu Ala Asp Arg Leu Val Arg Val His
   305                 310                 315                 320
   ggt gat cct gca gtg tgg tgg gta tcc cag ttt gtc aag tac ttg att   1008
   Gly Asp Pro Ala Val Trp Trp Val Ser Gln Phe Val Lys Tyr Leu Ile
                   325                 330                 335
   cgc cca caa ccc tgg ctg gaa aag gaa ata gaa gag gcc acc aag aag   1056
   Arg Pro Gln Pro Trp Leu Glu Lys Glu Ile Glu Glu Ala Thr Lys Lys
               340                 345                 350
   cta ggc ttc aaa cat cca gtt att gga gtc cat gtt aga cgc aca gac   1104
   Leu Gly Phe Lys His Pro Val Ile Gly Val His Val Arg Arg Thr Asp
           355                 360                 365
   aaa gtg gga gcg gaa gca gcc ttc cat ccc att gag gaa tac acg gtg    1152
   Lys Val Gly Ala Glu Ala Ala Phe His Pro Ile Glu Glu Tyr Thr Val
       370                 375                 380
   cac gtt gaa gaa gac ttt cag ctt ctt gct cgc aga atg caa gtg gat    1200
   His Val Glu Glu Asp Phe Gln Leu Leu Ala Arg Arg Met Gln Val Asp
   385                 390                 395                 400
   aaa aaa agg gtg tat ttg gcc aca gat gac cct gct ttg tta aaa gag    1248
   Lys Lys Arg Val Tyr Leu Ala Thr Asp Asp Pro Ala Leu Leu Lys Glu
                   405                 410                 415
   gca aaa aca aag tac ccc agt tat gaa ttt att agt gat aac tct atc    1296
   Ala Lys Thr Lys Tyr Pro Ser Tyr Glu Phe Ile Ser Asp Asn Ser Ile
               420                 425                 430
   tct tgg tca gct gga cta cat aat cga tat aca gaa aat tca ctt cgg    1344
   Ser Trp Ser Ala Gly Leu His Asn Arg Tyr Thr Glu Asn Ser Leu Arg
           435                 440                 445
   ggt gtg atc ctg gat ata cac ttt ctc tcc cag gca gac ttc cta gtg    1392
   Gly Val Ile Leu Asp Ile His Phe Leu Ser Gln Ala Asp Phe Leu Val
       450                 455                 460
   tgt act ttt tca tcg cag gtc tgt aga gtt gct tat gaa atc atg caa    1440
   Cys Thr Phe Ser Ser Gln Val Cys Arg Val Ala Tyr Glu Ile Met Gln
   465                 470                 475                 480
   gcg ctg cat cct gat gcc tct gcg aac ttc cgt tct ttg gat gac atc    1488
   Ala Leu His Pro Asp Ala Ser Ala Asn Phe Arg Ser Leu Asp Asp Ile
                   485                 490                 495
   tac tat ttt gga ggc cca aat gcc cac aac caa att gcc att tat cct    1536
   Tyr Tyr Phe Gly Gly Pro Asn Ala His Asn Gln Ile Ala Ile Tyr Pro
               500                 505                 510
   cac caa cct cga act gaa gga gaa atc ccc atg gaa cct gga gat att    1584
   His Gln Pro Arg Thr Glu Gly Glu Ile Pro Met Glu Pro Gly Asp Ile
           515                 520                 525
   att ggt gtg gct gga aat cac tgg gat ggc tat cct aaa ggt gtt aac    1632
   Ile Gly Val Ala Gly Asn His Trp Asp Gly Tyr Pro Lys Gly Val Asn
       530                 535                 540
   aga aaa ctg gga agg acg ggc cta tat ccc tcc tac aaa gtt cga gag    1680
   Arg Lys Leu Gly Arg Thr Gly Leu Tyr Pro Ser Tyr Lys Val Arg Glu
   545                 550                 555                 560
   aag ata gaa aca gtc aag tac ccc aca tat ccc gag gct gac aag taa    1728
   Lys Ile Glu Thr Val Lys Tyr Pro Thr Tyr Pro Glu Ala Asp Lys  *
                   565                 570                 575
<210> 14
   <211> 575
   <212> PRT
   <213> Sus scrofa
<400> 14
   Met Arg Pro Trp Thr Gly Ser Trp Arg Trp Ile Met Leu Ile Leu Phe
    1               5                  10                  15
   Ala Trp Gly Thr Leu Leu Phe Tyr Ile Gly Gly His Leu Val Arg Asp
               20                  25                  30
   Asn Asp His Ser Asp His Ser Ser Arg Glu Leu Ser Lys Ile Leu Ala
               35                  40                  45
   Lys Leu Glu Arg Leu Lys Gln Gln Asn Glu Asp Leu Arg Arg Met Ala
       50                  55                  60
   Glu Ser Leu Arg Ile Pro Glu Gly Pro Ile Asp Gln Gly Pro Ala Ser
   65                  70                  75                  80
   Gly Arg Val Arg Ala Leu Glu Glu Gln Phe Met Lys Ala Lys Glu Gln
                   85                  90                  95
   Ile Glu Asn Tyr Lys Lys Gln Thr Lys Asn Gly Pro Gly Lys Asp His
               100                 105                 110
   Glu Ile Leu Arg Arg Arg Ile Glu Asn Gly Ala Lys Glu Leu Trp Phe
           115                 120                 125
   Phe Leu Gln Ser Glu Leu Lys Lys Leu Lys Asn Leu Glu Gly Asn Glu
       130                 135                 140
   Leu Gln Arg His Ala Asp Glu Phe Leu Ser Asp Leu Gly His His Glu
   145                 150                 155                 160
   Arg Ser Ile Met Thr Asp Leu Tyr Tyr Leu Ser Gln Thr Asp Gly Ala
                   165                 170                 175
   Gly Asp Trp Arg Glu Lys Glu Ala Lys Asp Leu Thr Glu Leu Val Gln
               180                 185                 190
   Arg Arg Ile Thr Tyr Leu Gln Asn Pro Lys Asp Cys Ser Lys Ala Lys
           195                 200                 205
   Lys Leu Val Cys Asn Ile Asn Lys Gly Cys Gly Tyr Gly Cys Gln Leu
       210                 215                 220
   His His Val Val Tyr Cys Phe Met Ile Ala Tyr Gly Thr Gln Arg Thr
   225                 230                 235                 240
   Leu Ala Leu Glu Ser His Asn Trp Arg Tyr Ala Thr Gly Gly Trp Glu
                   245                 250                 255
   Thr Val Phe Arg Pro Val Ser Glu Thr Cys Thr Asp Arg Ser Gly Ser
               260                 265                 270
   Ser Thr Gly His Trp Ser Gly Glu Val Lys Asp Lys Asn Val Gln Val
           275                 280                 285
   Val Glu Leu Pro Ile Val Asp Ser Val His Pro Arg Pro Pro Tyr Leu
       290                 295                 300
   Pro Leu Ala Val Pro Glu Asp Leu Ala Asp Arg Leu Val Arg Val His
   305                 310                 315                 320
   Gly Asp Pro Ala Val Trp Trp Val Ser Gln Phe Val Lys Tyr Leu Ile
                   325                 330                 335
   Arg Pro Gln Pro Trp Leu Glu Lys Glu Ile Glu Glu Ala Thr Lys Lys
               340                 345                 350
   Leu Gly Phe Lys His Pro Val Ile Gly Val His Val Arg Arg Thr Asp
           355                 360                 365
   Lys Val Gly Ala Glu Ala Ala Phe His Pro Ile Glu Glu Tyr Thr Val
       370                 375                 380
   His Val Glu Glu Asp Phe Gln Leu Leu Ala Arg Arg Met Gln Val Asp
   385                 390                 395                 400
   Lys Lys Arg Val Tyr Leu Ala Thr Asp Asp Pro Ala Leu Leu Lys Glu
                   405                 410                 415
   Ala Lys Thr Lys Tyr Pro Ser Tyr Glu Phe Ile Ser Asp Asn Ser Ile
               420                 425                 430
   Ser Trp Ser Ala Gly Leu His Asn Arg Tyr Thr Glu Asn Ser Leu Arg
           435                 440                 445
   Gly Val Ile Leu Asp Ile His Phe Leu Ser Gln Ala Asp Phe Leu Val
       450                 455                 460
   Cys Thr Phe Ser Ser Gln Val Cys Arg Val Ala Tyr Glu Ile Met Gln
   465                 470                 475                 480
   Ala Leu His Pro Asp Ala Ser Ala Asn Phe Arg Ser Leu Asp Asp Ile
                   485                 490                 495
   Tyr Tyr Phe Gly Gly Pro Asn Ala His Asn Gln Ile Ala Ile Tyr Pro
               500                 505                 510
   His Gln Pro Arg Thr Glu Gly Glu Ile Pro Met Glu Pro Gly Asp Ile
           515                 520                 525
   Ile Gly Val Ala Gly Asn His Trp Asp Gly Tyr Pro Lys Gly Val Asn
       530                 535                 540
   Arg Lys Leu Gly Arg Thr Gly Leu Tyr Pro Ser Tyr Lys Val Arg Glu
   545                 550                 555                 560
   Lys Ile Glu Thr Val Lys Tyr Pro Thr Tyr Pro Glu Ala Asp Lys
                   565                 570                 575
<210> 15
   <211> 37
   <212> DNA
   <213> Artificial Sequence
<220>
   <223> PCR primer SH415
<400> 15
   ggcggccgcc accatggcac acgcaccggc acgctgc 37
<210> 16
   <211> 33
   <212> DNA
   <213> Artificial Sequence
<220>
   <223> PCR primer SH413
<400> 16
   ttaattaatc aggcattggg gtttgtcctc atg 33
<210> 17
   <211> 37
   <212> DNA
   <213> Artificial Sequence
<220>
   <223> PCR primer SH414
<400> 17
   ggcggccgcc accatgggtg aaccccaggg atccatg 37
<210> 18
   <211> 33
   <212> DNA
   <213> Artificial Sequence
<220>
   <223> PCR primer SH411
<400> 18
   ttaattaatc acttccgggc ctgctcgtag ttg 33
<210> 19
   <211> 36
   <212> DNA
   <213> Artificial Sequence
<220>
   <223> PCR primer RCD679
<400> 19
   gcggccgcca ccatgaatag ggcccctctg aagcgg 36
<210> 20
   <211> 32
   <212> DNA
   <213> Artificial Sequence
<220>
   <223> PCR primer RCD680
<400> 20
   ttaattaatc acacccccat ggcgctcttc tc 32
<210> 21
   <211> 36
   <212> DNA
   <213> Artificial Sequence
<220>
   <223> PCR primer SH420
<400> 21
   gcggcgcgcc gataatgacc accctgatca ctccag 36
<210> 22
   <211> 37
   <212> DNA
   <213> Artificial Sequence
<220>
   <223> PCR primer SH421
<400> 22
   ccttaattaa ctatttttca gcttcaggat atgtggg 37
<210> 23
   <211> 32
   <212> DNA
   <213> Artificial Sequence
<220>
   <223> PCR primer rEPO-forward
<400> 23
   gggaattcgc tcccccacgc ctcatttgcg ac 32
<210> 24
   <211> 32
   <212> DNA
   <213> Artificial Sequence
<220>
   <223> PCR primer rEPO-reverse
<400> 24
   cctctagatc acctgtcccc tctcctgcag gc 32
<210> 25
   <211> 46
   <212> DNA
   <213> Artificial Sequence
<220>
   <223> PCR primer ScMntI-forward
<400> 25
   gggcggccgc caccatggcc ctctttctca gtaagagact gttgag 46
<210> 26
   <211> 43
   <212> DNA
   <213> Artificial Sequence
<220>
   <223> PCR primer ScMnt1-reverse
<400> 26
   ccggcgcgcc cgatgacttg ttgttcaggg gatatagatc ctg 43